# EUROPEAN PATENT APPLICATION

(11) **EP 4 595 958 A1**
(43) Date of publication of application: **06.08.2025**
(21) Application number: 25154450.8
(22) Date of filing: 28.01.2025
(51) Int. Cl.: A61K 31/352, A61K 36/185, A61P 29/00

(54) **CANNABIS COMPOSITIONS ENRICHED WITH CBCA/CBC FOR CONSISTENT PAIN RELIEF**

(30) Priority: 29.01.2024 US 202463626117 P
(71) Applicant: Panaxia Pharmaceutical Industries Ltd., 7116002 Lod (IL)
(72) Inventor: KORNBLAU, Sarah, 9375401 Jerusalem (IL); LEVINTON, Genia, 9358805 Jerusalem (IL); HERSHKOVITS, Shany, 7316000 Mazor (IL); RAPOPORT, Anat, 6927530 Tel Aviv (IL); BARAK, Leehee, 6948320 Tel Aviv (IL); GOLDBERG, Eran, 5258210 Ramat Gan (IL); SEGAL, Dadi, 6473118 Tel Aviv (IL)
(74) Representative: Kancelaria Eupatent.pl Sp. z.o.o

(57) **Abstract**

Disclosed are cannabis compositions including CBCA and/or CBC at or above a predetermined minimal concentration that provides a consistent therapeutic effect, and methods of using the same, for treating pain.

## Description

### TECHNICAL FIELD

The present disclosure relates to cannabis compositions having a particular content of CBC/CBCA and use of the cannabis composition for the treatment of various conditions.

### BACKGROUND OF THE INVENTION

Cannabis has become more and more widespread in medical treatments and has shown to have various benefits.

The main cannabinoids found in cannabis are cannabidiol (CBD) and tetrahydrocannabinol (THC), however numerous other cannabinoids, and other unrelated compounds such as terpenoids and flavonoids, present in various amounts and ratios, are also found in cannabis, for example, the cannabinoid cannabichromene (CBC) and its acid form cannabichromenic acid (CBCA), the former generated as a result of the decarboxylation of CBCA, e.g. during smoking.

It is a well-known fact that the complexity of medical cannabis research and the efficiency of the resulting treatment with prescribed cannabis in providing relief, tends to vary from product to product, from strain to strain (which are taken sequentially or even simultaneously by patients) and even within the same strain purchased from the same grower. At times even a negative effect is obtained. The reason for this variability is related to diversity of the cannabis composition over time along with the different genetic characteristics of each plant and inconsistent growing conditions that affect the concentrations of THC and CBD, as well as of other less studied cannabis molecules present at lower concentration, and therefore patients are not receiving consistent treatment.

In accordance, there is a clear and urgent need to tailor medical cannabis treatment for different medical indications and symptoms, minimizing trial-and-error approach for strain selection and ensuring consistent, standardized, and effective treatment for the individual patient's needs.

Pain is a particularly common condition or symptom estimated to affect one of every five adults worldwide. It has sensory and emotional bases, and both chronic and acute pain may significantly impact an individual's quality of life, and lead to physical and emotional distress.

Pain can be categorized based on its etiology. At the nociceptive level, pain can arise from damaged tissue, including for example acute pain caused by cuts, burns in external tissue, pain that result from skeletomuscular / orthopedic injuries such as spinal herniated discs, pain associated with cancer, pain of headaches and migraines, or for example stomachaches and abdominal pain that may result from obstruction in internal organs, and inflammation. Neuropathic pain results from damage to the nervous system, either peripheral nerve damage as seen in conditions like diabetic neuropathy, or damage to the central nervous system (CNS) e.g., spinal cord injury. Chronic pain persists over time and may be nociceptive, neuropathic, or a combination thereof, it may be widespread as in fibromyalgia (FM), and often is associated with inflammatory conditions like rheumatoid arthritis.

Pain can often be managed effectively using medications. The choice of medication depends on the type and cause of pain. Common classes of medications may be used for pain management including Nonsteroidal Anti-Inflammatory Drugs (NSAIDs) and Corticosteroids for reducing pain of inflammation, inflammatory diseases and injury, opioids for severe acute and chronic pain, antidepressants and anticonvulsants for managing neuropathic pain, and muscle relaxants for alleviating muscle spasms, such as back pain.

Notably, medication alone is often not sufficient for comprehensive pain management. Other approaches, such as physical therapy, psychological interventions, and lifestyle modifications, are often integrated into a multimodal approach to address pain effectively while minimizing the risk of medication-related side effects or dependency, which are a major adverse effect of the more powerful pain relievers, such as the opioids, the anticonvulsants, and the muscle relaxants. Substantial evidence suggests cannabis is effective in treating acute pain and chronic pain in adults, patients often report that cannabis alleviates their pain.

It is suggested that the endocannabinoid system plays an important role in pain modulation. Binding to CB1 receptors, cannabinoids have a modulating effect on GABAergic and Glutamatergic transmission, while also influencing the hypothalamic pituitary adrenal (HPA) axis, immune system activation, and neuroplastic mechanisms. Importantly, an anxiolytic effect may be mediated through serotonergic effects of CBD via 5-HT1A receptor activation, and of THC via CB1 receptor agonism.

In some countries pain can serve as a basis for medical cannabis treatment, whether the pain emerges as a condition (e.g., fibromyalgia, migraines) or as a symptom (e.g., injury), and whether manifesting as primary condition/indication, or as a comorbidity.

Nevertheless, as previously stated there is a large variability in response to medical cannabis consumption, specifically for treating pain. While in some cases cannabis may be an effective aid that can relieve pain, as well as stressed body and mind, in other cases, regular use can contribute to impaired sleep and anxiety that inurn may worsen the pain.

This poses a major obstacle to the standardization and authorization of the use of cannabis in medical treatment, particularly the therapeutic potential of cannabis-based treatment in providing a consistent beneficial outcome of pain relief remains unclear.

There is, therefore, a need for identifying cannabis compositions having a particular content of cannabinoid, or another cannabis compound, that makes them suitable for steady, unvarying treatment of pain.

### SUMMARY OF THE INVENTION

The present disclosure provides cannabis compositions, and standardized cannabis extract, and methods of treatment including the same, comprising cannabis molecules, namely CBC/CBCA, at concentrations/amounts that advantageously and surprisingly were found to be efficacious for treating pain, by significantly alleviating the pain in a consistent and invariable manner, thereby providing steady effect even across populations of patients suffering from different types of pain, including orthopedic pain, neuropathic pain, spinal disk herniation pain, fibromyalgia (FM) pain, and pain of headaches .

In some embodiments, it is disclosed that as the CBC/CBCA dosage in the cannabis composition, or standardized extracts, increases above a certain amount the treatment of pain is more effective, while the alleviating effect is reduced in cannabis compositions, or standardized extracts, containing reduced amounts of CBCA or of its neutral form CBC, the latter generated as a result of the decarboxylation of CBCA, e.g. during smoking, extraction and distillation processes, and to a lesser extent also spontaneously.

According to some other embodiments, at least a minimal ratio between CBCA and/or CBC and THC concentrations is required for efficacious treatment. Additional embodiments, provides an advantageous and surprising combination between CBCA and Cannflavin A that was also found to correlate with treatment efficacy as the dosage of each of the molecules increases above its specified predetermined minimal amount/concentration.

Finally, according to another aspects, the disclosure provides methods of evaluating such compositions for having a particular content that makes them suitable for efficacious treatment, thereby the methods allow classifying and labeling these cannabis compositions for use in the treatment of pain.

To conclude, according to some embodiments, a cannabis composition/standardized extract of the invention may fulfil one or more characteristics required for it to be suitable for efficacious treatment of pain, including having (i) at least a predetermined minimal amount/concentration of CBCA and/or CBC per daily dose; (ii) CBCA and/or CBC to THC concentrations at least at the minimal characteristic ratio that was determined; (iii) enriched amount of CBCA and/or CBC of at least twice the predetermined minimal amount/concentration; and (iv) a combination of at least predetermined minimal amount of CBCA and/or CBC with at least a minimal amount of Cannflavin A.

According to one aspect, there is provided a cannabis composition comprising a predetermined minimal amount/concentration of at least 575ug CBCA and/or CBC per daily dose, for use in treatment of pain in a subject in need thereof.

In some embodiments, the predetermined minimal amount of CBCA and/or CBC is at least 800ug, per daily dose.

According to some additional embodiments, the predetermined minimal amount/concentration of CBCA and/or CBC is at least 1000ug, per daily dose.

In some related embodiments, the cannabis composition further comprises cannflavin A at an amount of at least 50ug, per daily dose.

In some embodiments, the pain comprises one or more type(s) of pain selected from orthopedic pain, neuropathic pain, spinal disk herniation pain, fibromyalgia (FM) pain, and headaches pain, or any combination thereof.

In some specific embodiments, the pain comprises headaches.

According to some embodiments, the composition comprises cannflavin A and total THC at concentrations characterized by cannflavin A to total THC ratio of at least about 0.07%.

In some related embodiments, the composition comprises CBCA and/or CBC and total THC concentrations characterized by CBCA and/or CBC to total THC ratio of at least about 0.8%.

In some other embodiments, the cannabis composition comprises raw cannabis.

In some embodiments, at least a portion of the CBCA and/or CBC in the cannabis composition comprises extracted, isolated, purified, and/or synthetic CBCA and/or CBC.

According to some other embodiments, the composition is selected from a plurality of potential cannabis compositions based on an analysis of the content of CBCA and/or CBC in the plurality of potential cannabis compositions.

According to some additional embodiments, CBCA and/or CBC is added to the composition if the CBCA and/or CBC content is below the predetermined minimal amount/concentration.

In some embodiments, the administering comprises administering by route of smoking, inhalation, vaporization, oral administration, buccal administration, nasal administration, topical administration, transdermal administration parenteral administration, or any combination thereof.

In some embodiments, the pain is characterized by a high level of perceived severity, and wherein the high level of perceived severity comprises self-reported ranking of 4 or 5 on a scale of 1-5.

According to some embodiments, the pain is associated with at least one additional medical condition.

According to some embodiments, the cannabis composition comprises a standardized extract.

According to some specific embodiments, the cannabis composition comprises a standardized extract comprising at least about 2.6% (w/w) THC, at least about 2.6% (w/w) CBD, at least about 0.1% (w/w) CBCA and/or CBC, and optionally 0.5% (w/w) Vitamin E.

In some embodiments, the cannabis composition comprises cannabis extracts enriched with CBCA and/or CBC.

In some specific embodiments, the cannabis composition enriched with CBCA and/or CBC comprises at least 1.1 times the predetermined minimal amount/concentration of CBCA and/or CBC.

In some embodiments, the cannabis composition comprises a content of CBCA and/or CBC between the predetermined minimal amount/concentration and a maximum amount/concentration of about 1.0g, per daily dose.

In some embodiments, the composition comprises CBCA and/or CBC at maximal concentration of less than 20.00%.

In some embodiments, the cannabis composition comprises cannabis extracts enriched with cannflavin A.

In some embodiments, the cannabis composition enriched with cannflavin A comprises at least 1.1 times the predetermined minimal amount/concentration of cannflavin A.

According to another aspect, there is provided a method for classifying cannabis compositions as being suitable for the treatment of pain, the method comprising:
receiving a potential cannabis composition;
evaluating the content of CBCA and/or CBC in the potential cannabis composition;
classifying the composition as being suitable for the treatment of pain when the CBCA and/or CBC content is at or above a predetermined minimal amount/concentration of 575ug, per daily dose.

In some embodiments, the predetermined minimal amount of CBCA and/or CBC is at least 800ug, per daily dose. In some embodiments, the predetermined minimal amount is at least 1000ug CBCA and/or CBC, per daily dose.

In some embodiments, the method further comprises evaluating the content of cannflavin A in the potential cannabis composition and classifying the composition as being suitable for the treatment of pain when the cannflavin A content is at or above a predetermined minimal amount/concentration of 50ug, per daily dose.

In some embodiments, the pain comprises one or more type(s) of pain selected from: orthopedic pain, neuropathic pain, spinal disk herniation pain, fibromyalgia (FM) pain, and headaches pain, or any combination thereof.

In some embodiments, the pain comprises headaches.

According to some embodiments, the method of classification comprises labeling the potential composition as being suitable for the treatment of pain, when the CBCA and/or CBC content is at or above the predetermined minimal amount/concentration.

According to some embodiments, the method of classification further comprises labeling the potential composition as being suitable for the treatment of headaches, when the CBCA and cannflavin A content is at or above the predetermined minimal amount/concentration.

According to an additional aspect, there is provided a standardized cannabis extract comprising extracts from a natural source and isolated and/or synthetic CBCA and/or CBC, wherein the extract comprises CBCA and/or CBC at a predetermined minimal amount/concentration of at least 575ug per daily dose.

In some specific embodiments, wherein the predetermined minimal amount of CBCA and/or CBC is at least 800ug, per daily dose. In some specific embodiments, the predetermined minimal amount/concentration of CBCA and/or CBC is at least 1000ug, per daily dose.

In some related embodiments, the extract further comprises cannflavin A at an amount of at least 50ug per daily dose.

According to some embodiments, the standardized extract comprises cannflavin A and total THC at concentrations characterized by cannflavin A to total THC ratio of at least about 0.07%.

In some embodiments, the standardized extract comprises CBCA and/or CBC and total THC at concentrations characterized by a CBCA and/or CBC to total THC ratio of at least about at least about 0.8%.

In some embodiments, the standardized extract comprises: at least about 2.6% (w/w) total THC, at least about 2.6% (w/w) total CBD, at least about 0.1% (w/w) CBCA and/or CBC and optionally about 0.5% (w/w) Vitamin E.

In some additional embodiments, the cannabis extract is enriched with CBCA and/or CBC; and wherein the enriched cannabis extract comprises at least about 1.1 times the predetermined minimal amount/concentration of CBCA and/or CBC.

In some embodiments, the standardized extract comprises a CBCA and/or CBC amount between the predetermined minimal amount and a maximum amount of about 1.0g, per daily dose.

In some embodiments, the standardized extract comprises CBCA and/or CBC at maximal concentration of less than 20.00%.

According to yet another aspect, there is provided a method for treating pain in a subject in need thereof, the method comprises administering to the subject a therapeutically effective amount of a cannabis composition comprising CBCA and/or CBC at or above a predetermined minimal amount/concentration of 575ug, per daily dose.

In some specific embodiments, the predetermined minimal amount is at least 800 ug CBCA and/or CBC per daily dose. In some specific embodiments, the predetermined minimal amount is at least 1000 ug CBCA and/or CBC, per daily dose.

In some related embodiments, the cannabis composition further comprises cannflavin A at an amount of at least 50ug, per daily dose.

According to some embodiments, the pain comprises one or more type(s) of pain selected from orthopedic pain, neuropathic pain, spinal disk herniation pain, fibromyalgia (FM) pain, and headaches pain, or any combination thereof.

In some embodiments, the pain comprises headaches.

In some embodiments, the administering comprises administering by route of smoking, inhalation, vaporization, oral administration, buccal administration, nasal administration, topical administration, transdermal administration parenteral administration, or any combination thereof.

In some embodiments, the pain is characterized by a high level of perceived severity, and wherein the high level of perceived severity comprises self-reported ranking of 4 or 5 on a scale of 1-5.

In some embodiments, the pain is associated with at least one additional medical condition.

Certain embodiments of the present disclosure may include some, all, or none of the above advantages. One or more technical advantages may be readily apparent to those skilled in the art from the figures, descriptions, and claims included herein.

In addition to the exemplary aspects and embodiments described above, further aspects and embodiments will become apparent by reference to the figures and by study of the following detailed descriptions.

### BRIEF DESCRIPTION OF THE FIGURES

The invention will now be described in relation to certain examples and embodiments with reference to the following illustrative figures.

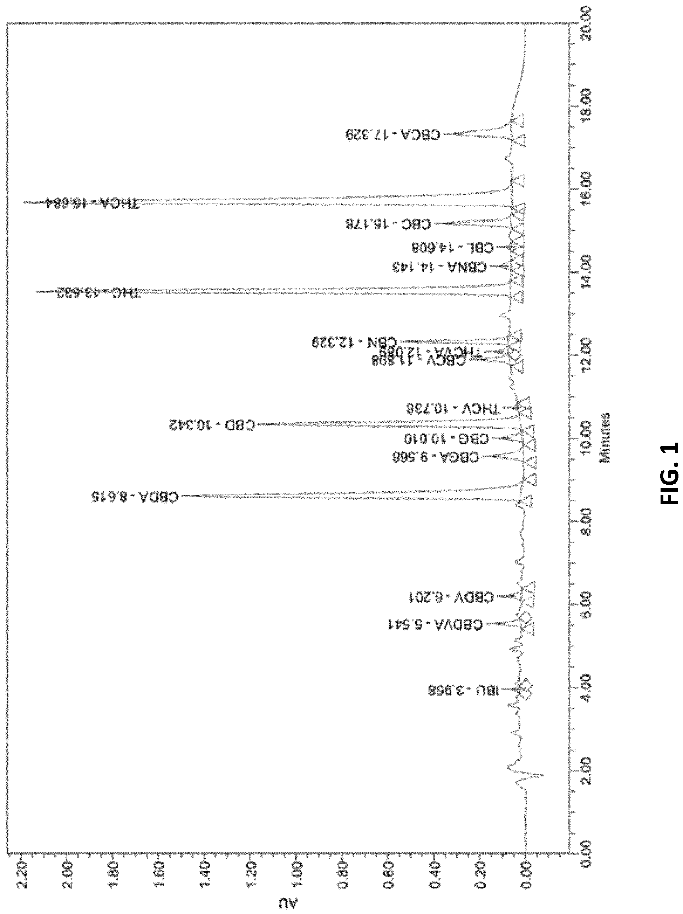

**FIG. 1** shows a typical chromatogram of the specific HPLC method applied in this study, obtained at 235nm wavelength.

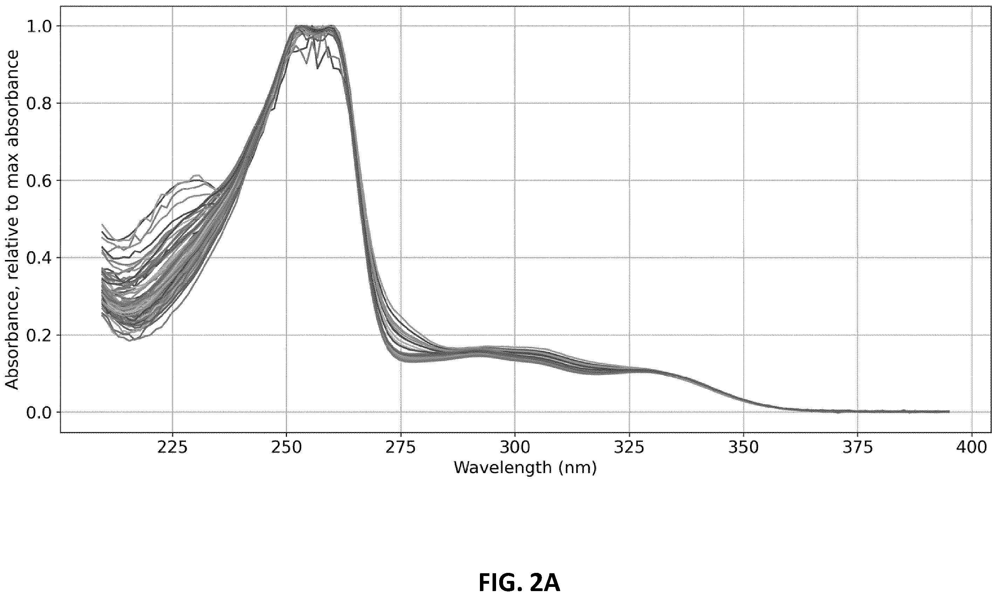

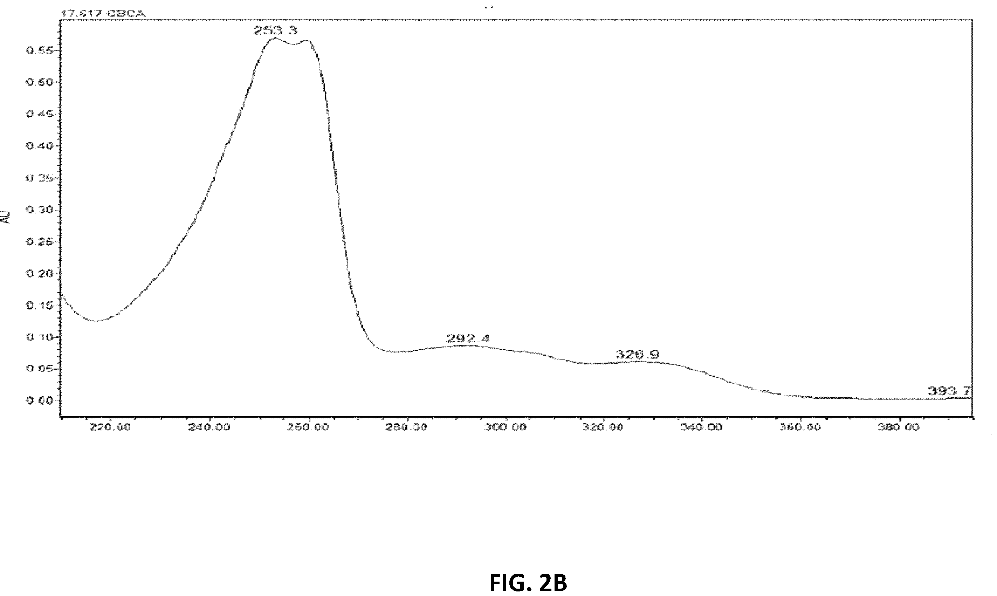

**FIGs. 2A-2B** show line graphs presenting the UV spectra identified as belonging to CBCA across samples obtained using HPLC (**FIG. 2A**) and the corresponding UV spectra obtained for CBCA standard (**FIG. 2B**).

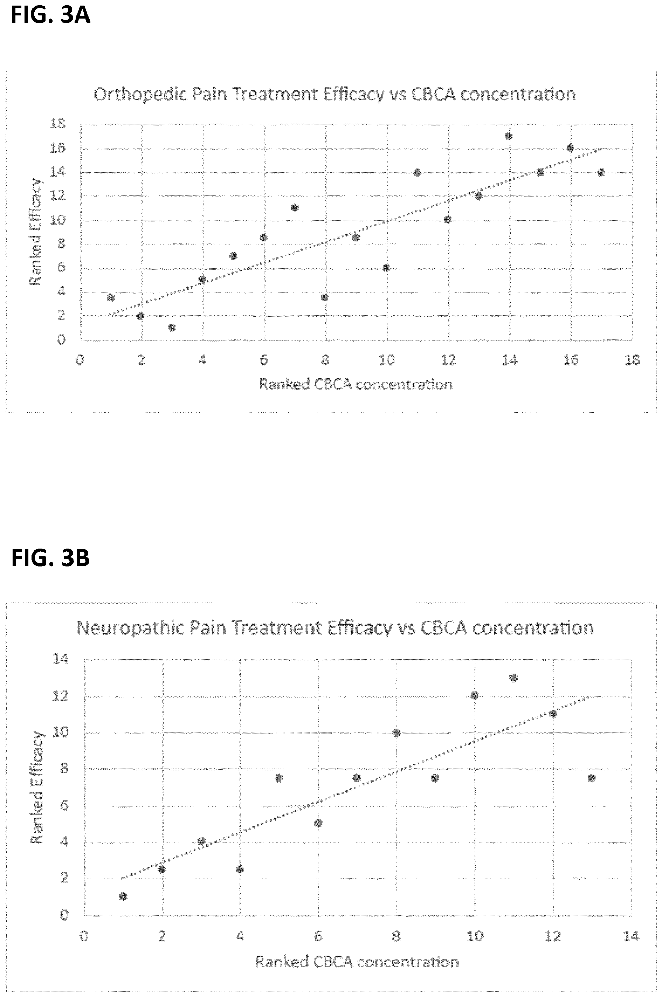

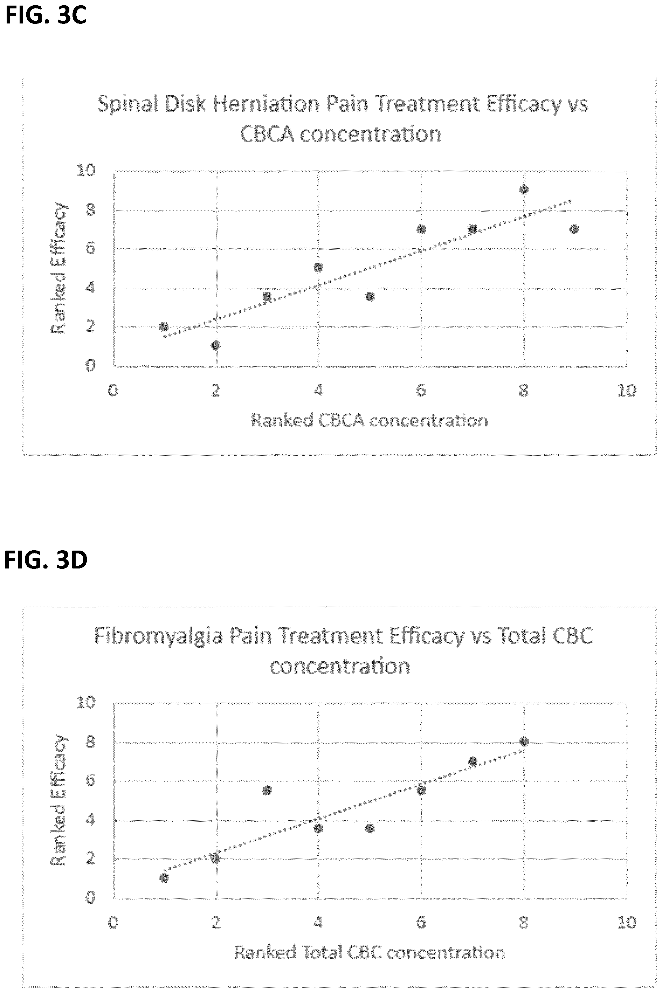

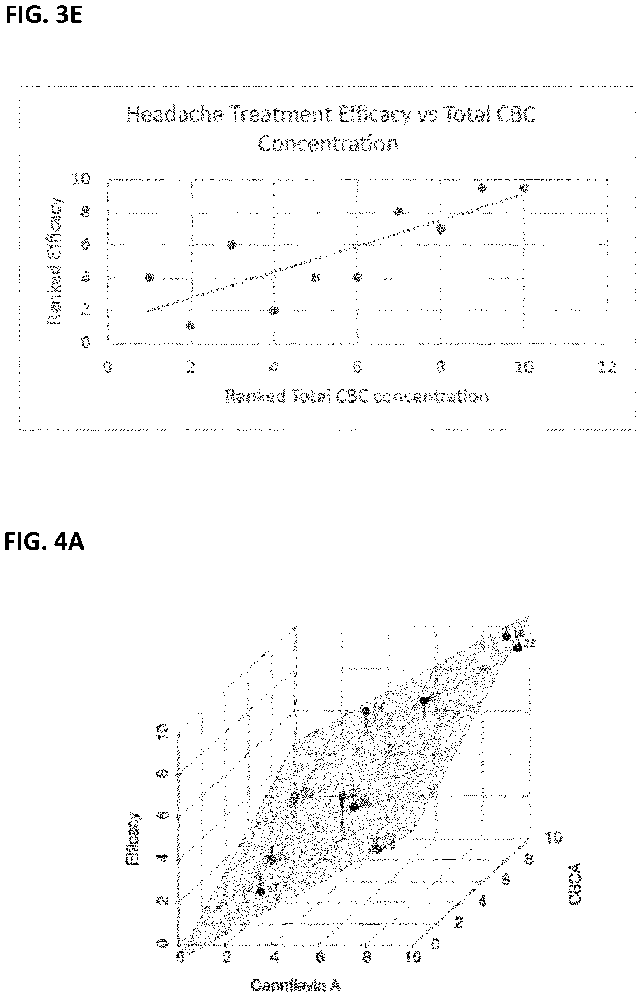

**FIGs. 3A-3E** show scatter plots presenting ranked treatment efficacy vs. ranked CBCA/Total CBC concentrations across strains/batches in the treatment of: orthopedic pain, r=0.87, FDR corrected p-value= 6.0E-05 (**FIG. 3A**, 17 strains); neuropathic pain, r=0.84, FDR corrected p-value= 2.7E-03 (**FIG. 3B**, 13 strains); spinal disk herniation pain, r=0.90, FDR corrected p-value= 1.2E-02 (**FIG. 3C**, 9 strains); fibromyalgia pain, r=0.89, FDR corrected p-value= 0.025 (**FIG. 3D**, 8 strains); headaches, r=0.81, p-value= 2.9E-02 (**FIG. 3E**, 10 batches). Each dot represents a strain/batch. All these p-values are after FDR multiple testing correction.

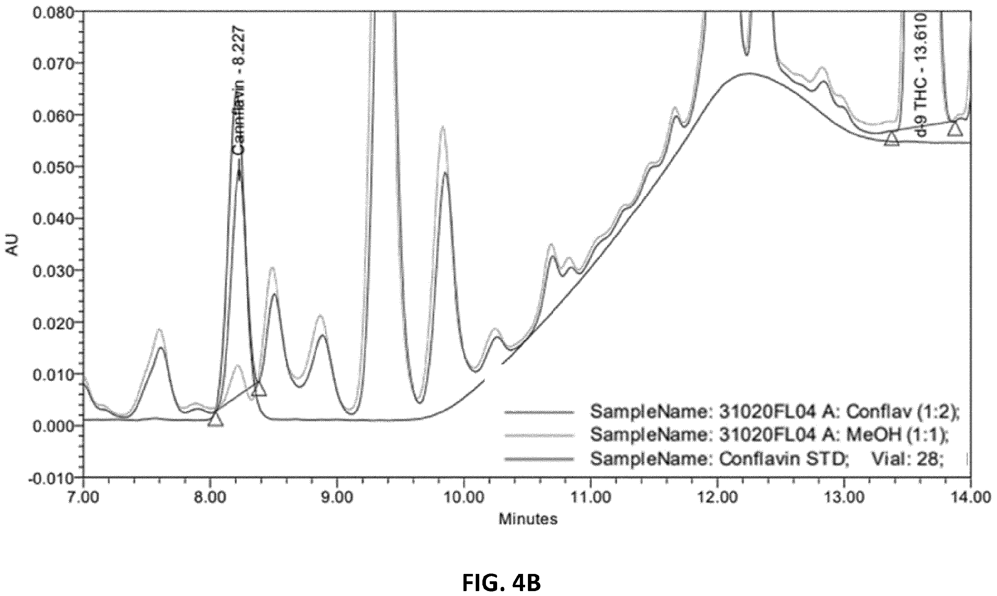

**FIG. 4A** show a scatter plot of a multiple linear regression model presenting ranked treatment efficacy of headaches vs. ranked concentrations of CBCA and Cannflavin A across 10 batches (**FIG. 4**). The regression model is illustrated by the pink plane. Each dot represents a strain/batch with its identification number. The differences of the predicted efficacies of each batch from their actual values are illustrated by the vertical red lines connecting the strains dots to the regression plane.
**FIG. 4B** shows a typical overlay of chromatograms of the specific HPLC method applied in this study (obtained at 235nm wavelength) presenting Cannflavin A standard (blue line), an inflorescence sample as-is (green line; ratio of 1:1 herbal substance: methanol) and the same inflorescence sample spiked with Cannflavin A standard (red line; ratio 1:2 herbal substance: cannflavin A.

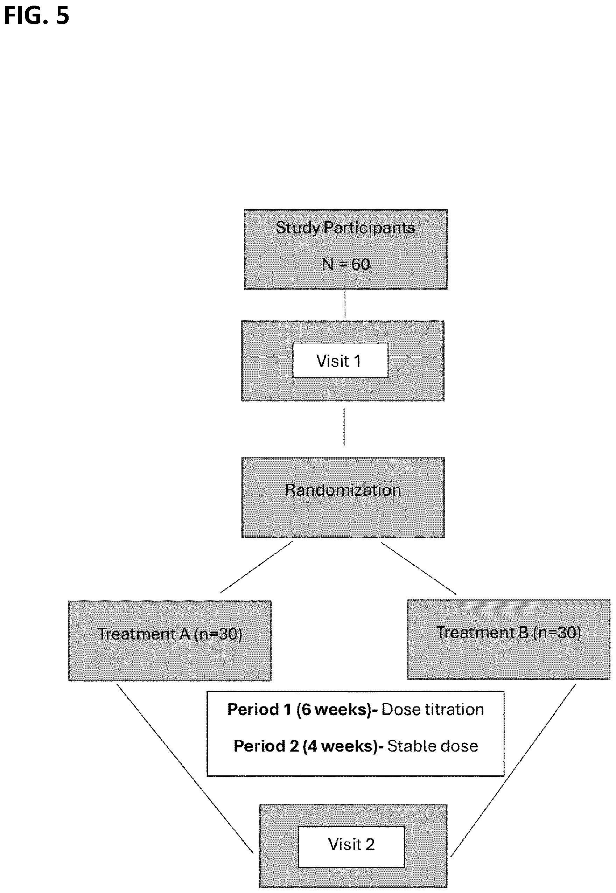

**FIG. 5** schematically illustrates the basic design principle of the clinical trial being performed to evaluate the effect of standardized cannabis extracts comprising at least the predetermined minimal amount/concentration of Cannflavin A and CBC, on subjects suffering from pain (Examples 7 and 8 describe clinical trial design with n=100 and n=150 patients, respectively). Period 1 is for evaluation of the baseline. Periods 2-3 is for evaluation of the combined CBC with Cannflavin A treatment against the placebo.

### DETAILED DESCRIPTION

In the following description, various aspects of the disclosure will be described. For the purpose of explanation, specific configurations and details are set forth in order to provide a thorough understanding of the different aspects of the disclosure. However, it will also be apparent to one skilled in the art that the disclosure may be practiced without specific details being presented herein. Furthermore, well-known features may be omitted or simplified in order not to obscure the disclosure.

As used herein, the singular forms "a", "an", and "the" are intended to include the plural forms as well, unless the context clearly indicates otherwise.

As used herein, the term "about" when referring to a measurable value such as an amount, a temporal duration, and the like, is meant to encompass variations of ±20% or in some instances ±10%, or in some instances ±5%, or in some instances ±1%, or in some instances ±0.1% from the specified value, as such variations are appropriate to perform the disclosed methods.

As used herein, the terms "subject", "patient" or "individual" may be used interchangeably and generally refer to a human, although the methods of the invention are not necessarily limited to humans and should be useful in other mammals.

According to some embodiments, the term "**essentially devoid of**" may refer to a stated material as either entirely absent or present in a residual amount, such as less than 2%, or less than 1%, or less than 0.1% are present. Each possibility is a separate embodiment.

In some embodiments, the composition is essentially devoid of THC and/or CBD. Each possibility is a separate embodiment. In some embodiments, the composition comprises THC and/or CBD at residuals amounts of less than 2%. In some embodiments, the composition comprises THC and/or CBD at residuals amounts of less than 1%. In some embodiments, the composition comprises THC and/or CBD at residuals amounts of less than 0.1%.

As used herein, the term "**comprising**" is synonymous with the terms "including," "containing," or "characterized by," and is inclusive or open-ended i.e. does not exclude additional, unrecited elements. According to some embodiments, the term comprising may be replaced with the term with the term "consisting of" which excludes any element, step, or ingredient not specified in the claim. According to some embodiments, the term comprising may be replaced with the term "**consisting essentially of**" which limits the scope of a claim to the specified materials or steps "and those that do not materially affect the basic and novel characteristics" of the claimed invention.

In some embodiments, the composition consists essentially of CBCA and/or CBC. Each possibility is a separate embodiment. In some embodiments, the composition consists essentially of CBCA and/or CBC and cannflavin A. Each possibility is a separate embodiment. In some embodiments, the composition consists essentially of CBCA and cannflavin A. In some embodiments, the composition consists essentially of cannflavin A.

As used herein the terms "**CBCA and/or CBC**" and "**CBCA/CBC**" are interchangeable and when embodied should inherently include all possible combinations, as accepted (i.e., CBCA alone, CBCA and CBC (total CBC), and CBC alone).

As used herein somewhere the term "**total CBC**" refers to CBCA and CBC (calculated as the sum of CBCA*0.877 + CBC), therefore the term "CBCA and/or CBC" may refer to total CBC. In some embodiments, CBCA and/or CBC includes total CBC.

According to an aspect, there is provided a cannabis composition comprising a predetermined minimal amount/concentration of CBCA and/or CBC for use in the treatment of pain in a subject in need thereof. Each possibility is a separate embodiment.

In some embodiments thereof, the predetermined minimal amount/concentration of CBCA and/or CBC is at least 575ug cannflavin, per daily dose; in some embodiments, the predetermined minimal amount/concentration of CBCA and/or CBC is at least 800ug CBCA and/or CBC, per daily dose. Each possibility is a separate embodiment.

In other embodiments, the predetermined minimal amount/concentration of CBCA and/or CBC is at least 1000ug cannflavin, per daily dose; in some additional embodiments, the predetermined minimal amount/concentration of CBCA and/or CBC is at least 1300ug CBCA and/or CBC, per daily dose. Each possibility is a separate embodiment.

In some embodiments, according to another aspect of the disclosure, there is provided a method for treating pain/pain-related condition and/or symptom in a subject in need thereof, the method comprising: selecting, from a plurality of optional cannabis compositions, a suitable cannabis composition, the suitable cannabis composition characterized by containing a predetermined minimal amount/concentration of CBCA and/or CBC and administering a therapeutically effective amount of the suitable CBCA and/or CBC composition to the subject.

As used herein, the term **"treating"** or "treatment" refers to an approach for obtaining beneficial or desired results, including clinical results. Beneficial or desired clinical results can include, but are not limited to, alleviation or amelioration of one or more symptoms or conditions (i.e., pain), diminishment of the extent of a disease or of a condition and/or symptom, stabilization of the state of a disease or a condition and/or symptom, prevention of deterioration of the disease or condition, delay or slowing of disease progression, amelioration or palliation of the condition and/or symptom state, and remission (whether partial or total). The terms "disease", "condition" and "indication" may be interchangeably used.

As used herein, the terms "prevent", "alleviate", "reduce", "attenuate", "ameliorate", "inhibit" are used interchangeably.

The term **"pain",** has the meanings normally ascribed to it in the art, and as used herein refers to any type of pain whether it is a persistent pain that lasts for an extended period or to an acute pain, and whether manifested as a condition characterized by pain (e.g., fibromyalgia (FM)) or as an associated symptom (e.g., orthopedic pain), and including, in some embodiments, acute pain and chronic pain, nociceptive pain and neuropathic pain, such as but not limited to orthopedic pain, neuropathic pain, spinal disc herniation pain, fibromyalgia (FM) pain, headache pain, abdominal pain, widespread pain, or skeleto-muscle pain, and the like.

The term "pain" may more specifically refer to the indications for which treatment efficacy was herein exemplified to positively correlate with concentrations of CBCA and/or CBC and/or CBCA with cannflavin A, and including at least one of any one of orthopedic pain, neuropathic pain, spinal disc herniation pain, fibromyalgia (FM) pain, and/or headache pain, or any combination thereof.

In some embodiments, pain refers to persistent pain/chronic pain that lasts for an extended period or to an acute pain, each separately and combined. Each possibility is a separate embodiment.

The term "pain-related/associated **condition and/or symptom"** as used herein refers to indications characterized by pain (e.g., headaches including migraines) or indications associated with pain (e.g., spinal disk herniation pain). The terms "pain" "type(s) of pain" or "pain related/associated condition and/or symptom" are synonymous and can be used interchangeably.

In some embodiments, the pain comprises pain-related condition(s) and/or symptom(s).

In some embodiments, the pain comprises one or more type(s) of pain selected from orthopedic pain, neuropathic pain, spinal disc herniation pain, fibromyalgia (FM) pain, headache pain, abdominal pain, widespread pain, or skeleto-muscle pain, or any combination thereof. Each possibility is a separate embodiment.

In some embodiments, the pain comprises one or more type(s) of pain selected from orthopedic pain, neuropathic pain, spinal disc herniation pain, fibromyalgia (FM) pain, and headache pain, or any combination thereof. Each possibility is a separate embodiment.

In some embodiments, the pain comprises one or more type(s) of pain selected from orthopedic pain, neuropathic pain, spinal disc herniation pain.

In some embodiments, the pain comprises one or more type(s) of pain selected from fibromyalgia (FM) pain. In some embodiments, FM comprises widespread pain and/or skeleto-muscle pain.

In some embodiments, the pain comprises one or more type(s) of pain selected from headaches. In some embodiments, headaches comprise migraines.

As used herein, the term "**selecting**" or "selection" may refer to choosing from a plurality of options or choosing a product according to it fulfilling a certain requirement, here a minimal cannflavin concentration. According to some embodiments, the selection comprises selecting according to a product specification that outlines the amount/concentration of the cannabinoids or other compounds found in the product. According to some embodiments, the selecting comprises measuring/evaluating the concentration of the cannabis-related compound, for example using HPLC. According to some embodiments, the selecting comprises selecting a composition having a suboptimal concentration/amount of a desired cannabinoid, here CBC/CBCA, and adding to the composition the CBCA and/or CBC (for example, by adding isolated and/or synthetic CBCA and/or CBC) so as to achieve the desired concentration/amount.

As used herein, the term **"plurality"** may refer to two or more, three or more, five or more, or ten or more. Each possibility is a separate embodiment.

As used herein, the term **"cannabis composition"** may broadly refer to any product having cannabis as a major constituent. According to some embodiments, the cannabis composition may be a cannabis product suitable for medical use (medical grade). According to some embodiments, the cannabis composition comprises CBC/CBCA, THC and/or CBD as well as one or more additional cannabinoid or other compounds, such as terpenoids and flavonoids including Cannflavin A. According to some embodiments, the cannabis composition may be raw cannabis, such as buds, inflorescence, or resins. According to some embodiments, the cannabis composition may be cannabis extract or cannabis oil. According to some embodiments, the cannabis composition may include standardized extracts. According to some embodiments, the cannabis composition may be a composition (e.g., a pharmaceutical composition) including one or more isolated CBCA and/or CBC (in addition to THC and/or CBD). According to some embodiments, the composition may be in the form of a liquid, a powder, a tablet, a capsule or any other suitable form.

According to some embodiments, the cannabis composition may include one or more of natural CBCA/CBC, extracted CBCA/CBC, isolated CBCA/CBC, purified CBCA/CBC, and/or synthetic CBCA/CBC, or any combination thereof. Each possibility is a separate embodiment.

As used herein the term "**optional cannabis composition**" may refer to a line of actual cannabis products/composition/standardized formulations/extracts between which one can choose. Alternatively, the optional cannabis compositions may be theoretical compositions (e.g. all cannabis composition known and/or available).

As used herein the term "**suitable cannabis composition**" refers to a cannabis composition which can be chosen for use because it fulfils certain requirements that are not fulfilled by all the potential cannabis compositions (whether actual such as the herein disclosed predetermined minimal amount or theoretical). In some embodiments, the suitable cannabis composition comprises at least a predetermined minimal amount/concentration of CBCA and/or CBC per daily dose.

The cannabis composition of the present invention is made of cannabis extract.

In some embodiments, the cannabis composition of the present invention includes a standardized cannabis extract.

The terms "standardized cannabis extract" and "formulation" are synonymous and may refer to a "medical cannabis product", and may all refer to the cannabis composition of the present disclosure.

As used herein, the term "standardized extract", and "standardized cannabis extract" may be interchangeably used and refer to a cannabis extract from a natural source modified/formulated/standardized preferably to include (by addition) at least a certain amount/content of one or more active ingredient, such as but not limited to: cannabinoids like CBCA and/or CBC, THC, CBD, or cannflavin A, nevertheless according to less favorable instances of the invention the standardized extract may also be formulated without THC and/or CBD (possibly by excluding them or by using cannabis extracts having poor THC and/or CBD content).

The term "**cannflavin"** refers to a group/family of chemical compounds found in cannabis sativa and includes cannflavin A, cannflavin B, and cannflavin C. As used herein, the term "cannflavin" refers only to cannflavin A. Cannflavin A may include natural and synthetic cannflavin A.

According to some embodiments, the cannflavin is Cannflavin A.

According to some embodiments, the cannabinoid or cannflavin in the composition of the present invention may be derived from an extract including the cannabinoid or cannflavin, and/or may be a purified, or distilled/isolated or synthetic form thereof, or any combination thereof.

The term "**cannabinoid**" refers hereinafter to a class of diverse chemical compounds that act on cannabinoid receptors present in cells that repress neurotransmitter release in the brain. Cannabinoid compounds may include the endocannabinoids (produced naturally in the body by humans and animals), the phytocannabinoids (found in cannabis and some other plants), and synthetic cannabinoids. According to some embodiments, the at least one cannabinoid of the composition may be in the form of an extract including the cannabinoid, a purified or distilled cannabinoid, or combination of cannabinoids derived from an extract, or a cannabinoid or combination of cannabinoids synthetically produced.

As used herein, the term "**synthetic**" refers to a chemically synthesized CBCA/CBC or cannflavin molecule.

As used herein, the term "**extracted**" or "extract" refers to a molecule extracted from a natural source (i.e., plant source) and may be further added to the mixture/composition/formulation. As a non-limiting example, an extracted CBCA/CBC or cannflavin molecule may also include additional components (e.g. additional terpenoids, flavonoids or cannabinoids), which are added to the composition, but which are not necessarily required as part of the plant/extract used in the composition.

The extracted molecule(s) may be a cannabis plant extract, or it may be extracted from other plant source, including a plant genetically modified to include/express the molecule(s). The extracted molecule(s) may be used as the main extract for the production of cannabis composition/formulation of the present invention, or the extracted molecule(s) may be added to the cannabis composition/formulation/extract of the present invention.

As used herein, the term **"isolated"** refers to CBCA/CBC or cannflavin molecules which are extracted from a natural resource, so as to become a major component of the extracted mixture. The extracted mixture may also include smaller amounts of other molecules. As a non-limiting example, in some embodiments, isolated CBCA/CBC refers to an extract of mixture containing mostly CBCA/CBC, e.g. at least about 50%, at least about 60%, at least about 70% at least about 80% or at least about 90% CBCA/CBC. Each possibility is a separate embodiment.

As used herein, the term **"purified"** refers to CBCA/CBC or cannflavin molecules which are isolated from a natural source, and purified so as to become the essentially only component, i.e. other components are present in residual amounts only. As a non-limiting example, in some embodiments, purified CBCA/CBC refers to an extract containing essentially only CBCA/CBC, e.g. less than about 10% or less than about 5% less than about 2% of other molecules or less than about 1% of other molecules. Each possibility is a separate embodiment.

As used herein, the term **"administering"** includes routes of administration which allow the compositions of the invention to perform their intended function. A variety of routes of administration are possible including, but not necessarily limited to, smoking, vaporization, inhalation, nasal and/or buccal spraying, drops administered orally or nasally, oral administration (e.g., of tablets or capsules), topical administration, transdermal administration, parenteral administration or the like.

According to some embodiments, the cannabis composition may include a physiologically acceptable vehicle or carrier and optional excipients, adjuvants, and preservatives suitable for the route of administration. For solutions or emulsions or solid oral dosage forms (e.g., of tablets or capsules or caplets), suitable carriers include, for example, aqueous or alcoholic/aqueous solutions, emulsions or suspensions, including saline and buffered media, sterile water, creams, ointments, lotions, oils, pastes and solid carriers.

According to some embodiments, the herein disclosed cannabis composition comprises a predetermined minimal amount/concentration of CBCA and/or CBC.

It was found that the ranked median concentrations of CBCA/total CBC across medical cannabis strains and batches were positively correlated in a statistically significant manner with the strains' efficacy rankings provided by patients with orthopedic pain, neuropathic pain, spinal disk herniation pain, fibromyalgia pain, and headaches pain.

As used herein, the term **"predetermined minimal amount/concentration"** refers to the minimal content of CBCA and/or CBC herein found, and exemplified throughout **Examples 1-6,** to positively correlate with efficacious treatment of pain. These examples demonstrate that the consumption of cannabis composition and standardized extracts comprising predetermined minimal amount/concentration of CBCA and/or CBC not only aid by strongly reducing the perceived level of pain reported by the patients, but further contribute to consistent, steady, unvarying therapeutic effect.

According to some embodiments, efficacious treatment comprises reduction in pain. According to some related embodiments, efficacious treatment comprises consistent, unvarying therapeutic effect.

The predetermined amount/concentration of CBCA and/or CBC is the minimal amount required for efficacious treatment and was set, in some embodiments, as: a minimal dose of at least 575 ug/day determined based on the 50% percentile, which corresponds to 0.17% (w/w), and considering minimal consumption of 0.333 grams /per day by a subject (i.e., about 10 g per month); or in other some embodiments, as: a minimal dose of at least 800ug/day determined based on the 75% percentile, which corresponds to 0.24% (w/w), and considering minimal consumption of 0.333 grams/per day by a subject (i.e., about 10g per month).

The predetermined amount/concentration of CBCA and/or CBC is the minimal amount required for efficacious treatment and was set, in some embodiments, as: a minimal dose of at least 1000 ug/day determined based on the 50% percentile, which corresponds to 0.3% (w/w), and considering minimal consumption of 0.333 grams /per day by a subject (i.e., about 10 g per month); or in other some embodiments, as: a minimal dose of at least 1300ug/day determined based on the 75% percentile, which corresponds to 0.4% (w/w), and considering minimal consumption of 0.333 grams/per day by a subject (i.e., about 10g per month).

According to some embodiments, the cannabis composition comprises a predetermined minimal amount of CBCA and/or CBC for use in the treatment of pain/pain type(s) selected from **Table 1.**

In some specific embodiments, the cannabis composition comprises a predetermined minimal amount/concentration of CBCA and/or CBC of at least 575 ug/day, or at least 800 ug/day, or at least 1000 ug/day, or at least 1300 ug/day for use in the treatment of pain; wherein the pain comprises one or more type(s) of pain selected from orthopedic pain, neuropathic pain, spinal disk herniation pain, fibromyalgia (FM) pain, and headaches pain, or any combination thereof. Each possibility is a separate embodiment.

In some specific embodiments, the cannabis composition comprises a predetermined minimal amount/concentration of CBCA and/or CBC of at least 575 ug/day, or at least 800 ug/day for use in the treatment of pain; wherein the pain comprises one or more type(s) of pain selected from orthopedic pain, neuropathic pain, spinal disk herniation pain, and fibromyalgia (FM) pain, or any combination thereof. Each possibility is a separate embodiment.

In some specific embodiments, the cannabis composition comprises a predetermined minimal amount/concentration of CBCA and/or CBC of at least 1000 ug/day, or at least 1300 ug/day for use in the treatment of pain; wherein the pain comprises headaches. Each possibility is a separate embodiment.

In some additional specific embodiments, the cannabis composition comprises a predetermined minimal amount/concentration of CBCA of at least 575 ug/day, or at least 800 ug/day for use in the treatment of pain; wherein the pain comprises one or more type(s) of pain selected from orthopedic pain, neuropathic pain, and spinal disk herniation pain, or any combination thereof. Each possibility is a separate embodiment. Reference is now made to

### Examples 1-3.

In some additional specific embodiments, the cannabis composition comprises a predetermined minimal amount/concentration of CBCA and CBC (total CBC) of at least 575 ug/day, or at least 800 ug/day for use in the treatment of pain; wherein the pain comprises fibromyalgia (FM) pain. Each possibility is a separate embodiment. Reference is now made to

### Examples 4.

In some additional specific embodiments, the cannabis composition comprises a predetermined minimal amount/concentration of CBCA and CBC (total CBC) of at least 1000 ug/day, or at least 1300 ug/day for use in the treatment of pain; wherein the pain comprises headaches pain. Each possibility is a separate embodiment. Reference is now made to

### Examples 5.

In some additional specific embodiments, the cannabis composition comprises a predetermined minimal amount/concentration of CBCA of at least 1000 ug/day, or at least 1300 ug/day for use in the treatment of pain; wherein the pain comprises headaches pain. Each possibility is a separate embodiment. Reference is now made to **Examples 6.**

In some additional specific embodiments, the cannabis composition comprises a predetermined minimal amount/concentration of cannflavin A of at least 50 ug/day, or at least 60 ug/day for use in the treatment of pain; wherein the pain comprises headaches pain. Each possibility is a separate embodiment. Reference is now made to **Examples 6.**

In some additional specific embodiments, the cannabis composition comprises a predetermined minimal amount/concentration of CBCA of at least 1000 ug/day, or at least 1300 ug/day, and may further comprise cannflavin A at a minimal concentration of at least 50 ug/day or at least 60 ug/day for use in the treatment of pain; wherein the pain comprises headaches pain. Each possibility is a separate embodiment. Reference is now made to **Examples 6.**

Reference is now made to **Table 1.**

In some embodiments, the cannabis composition comprises a predetermined minimal amount/concentration of CBCA and/or CBC for use in the treatment of pain.

In some embodiments, the cannabis composition comprises a predetermined minimal amount/concentration of cannflavin A for use in the treatment of pain.

In some embodiments, the cannabis composition comprises a predetermined minimal amount/concentration of CBCA and/or CBC and a minimal amount/concentration of cannflavin A for use in the treatment of pain.

The predetermined minimal amount of CBCA and/or CBC may be any dose of CBCA and/or CBC (ug/day) determined based on, and corresponding to, at least the 50% percentile, as it was determined in **Examples 1-6.**

In some embodiments, the predetermined minimal amount of CBCA and/or CBC includes any dose of CBCA and/or CBC (ug/day) determined based on, and corresponding to: at least the 50% percentile, at least the 55% percentile, at least about the 60% percentile, at least about the 65% percentile, at least about the 70% percentile, at least about the 75% percentile, at least about the 80% percentile, at least about the 85% percentile, at least about the 90% percentile, at least about the 95% percentile, at least about the 99% percentile, at least about the 100% percentile, at least about the 115% percentile at least about the 130% percentile, or more. Each possibility is a separate embodiment.

In some embodiments, the predetermined minimal amount of CBCA and/or CBC may be between the 50% percentile and the 100% percentile, corresponding to 575ug/day and 1330ug/day, respectively.

In some embodiments, the predetermined minimal amount of CBCA and/or CBC may be between the 50% percentile and the 100% percentile, corresponding to 1000ug/day and 1730ug/day, respectively.

In some embodiments, the predetermined minimal amount of CBCA and/or CBC may include for example, but is not limited to, CBCA and/or CBC amount/concentrations that are determined based, and corresponding to, ranges between about the 50% percentile and the about 95% percentile, between about the 50% percentile and the about 90% percentile, between about the 50% percentile and the about 85% percentile, or between about the 50% percentile and the about 80% percentile. Each possibility is a separate embodiment.

In some embodiments, the cannabis composition further comprises cannflavin A at an amount of at least 50ug, per daily dose. In some embodiments, the cannabis composition further comprises cannflavin A at an amount of at least 60ug, per daily dose.

The predetermined minimal amount of cannflavin A may be any dose of cannflavin A (ug/day) determined based on, and corresponding to, at least the 50% percentile, as it was determined in **Examples 6.**

In some embodiments, the predetermined minimal amount of cannflavin A includes any dose of cannflavin A (ug/day) determined based on, and corresponding to: at least the 50% percentile, at least the 55% percentile, at least about the 60% percentile, at least about the 65% percentile, at least about the 70% percentile, at least about the 75% percentile, at least about the 80% percentile, at least about the 85% percentile, at least about the 90% percentile, at least about the 95% percentile, at least about the 99% percentile, at least about the 100% percentile, or more. Each possibility is a separate embodiment.

In some embodiments, the predetermined minimal amount of cannflavin A may be between the 50% percentile and the 100% percentile, corresponding to 50ug/day and 100ug/day, respectively.

Nevertheless, the predetermined amount/concentration defines the minimal threshold of CBCA and/or CBC and/or cannflavin A concentrations found to be essential for efficacious treatment having consistent and steady therapeutic effect for various conditions and/or symptoms across a wide range of patients, and it should not be considered as limiting the content of CBCA and/or CBC and/or cannflavin A in the disclosed compositions and standardized extracts of the invention.

Accordingly, it is described herein below that, in some embodiment, the cannabis composition comprises a content of CBCA and/or CBC of up to about 1,000,000ug (1.0g), per daily dose, or in other words, in some embodiment, the cannabis composition comprises a content of CBCA and/or CBC between the predetermined minimal amount/concentration and a maximum amount/concentration of about 1.0g, per daily dose.

The cannabis composition of the invention comprising a certain predetermined minimal amount/concentration that may be required to be consumed at cumulative amounts during the day in order for them to reach (i.e., administer the subject with) the predetermined minimal amount/concentration per daily dose of composition/product, thereby facilitating the benefits of the treatment that consistently alleviate/attenuate several pain-associated conditions and/or symptoms.

The terms "predetermined amount/concentration" and "predetermined minimal amount/concentration" may be interchangeably used.

According to some embodiments, the predetermined minimal amount of CBCA and/or CBC includes at least about 575ug of CBCA and/or CBC per daily dose unit, at least about 700ug of CBCA and/or CBC per daily dose unit, at least about 800ug of CBCA and/or CBC per daily dose unit, at least about 900ug of CBCA and/or CBC per daily dose unit, at least about 1000ug of CBCA and/or CBC per daily dose unit, at least about 1100ug of CBCA and/or CBC per daily dose unit, at least about 1200ug of CBCA and/or CBC per daily dose unit, at least about 1300ug of CBCA and/or CBC per daily dose unit, at least about 1400ug of CBCA and/or CBC per daily dose unit, at least about 1500ug of CBCA and/or CBC per daily dose unit, at least about 1600ug of CBCA and/or CBC per daily dose unit, at least about 1700ug of CBCA and/or CBC per daily dose unit, at least about 1800ug of CBCA and/or CBC per daily dose unit, at least about 2200ug of CBCA and/or CBC per daily dose unit, at least about 2500ug of CBCA and/or CBC per daily dose unit Each possibility is a separate embodiment.

According to some embodiments, the predetermined minimal amount of CBCA and/or CBC comprises between about 575ug and about 3000ug of CBCA and/or CBC per daily dose unit, or between about 575ug and about 1800ug of CBCA and/or CBC per daily dose unit.

According to some embodiments, the predetermined minimal amount of CBCA and/or CBC comprises between about 575ug and about 1400ug of CBCA and/or CBC per daily dose unit, or between about 800ug and about 1400ug of CBCA and/or CBC per daily dose unit. Each possibility is a separate embodiment.

According to some embodiments, the predetermined minimal amount of CBCA and/or CBC comprises between about 1000ug and about 3000ug of CBCA and/or CBC per daily dose unit, or between about 1000ug and about 1800ug of CBCA and/or CBC per daily dose unit, or between about 1300ug and about 1800ug of CBCA and/or CBC per daily dose unit. Each possibility is a separate embodiment.

According to some embodiments, the cannabis composition or the cannabis extract comprises at least a predetermined minimal amount and is limited by the maximum amount/concentration of about 1.0g per daily dose, or less. Each possibility is a separate embodiment.

According to some embodiments, the predetermined minimal amount of cannflavin includes at least about 50ug of cannflavin per daily dose unit, at least about 55ug of cannflavin per daily dose unit, at least about 60ug of cannflavin per daily dose unit, at least about 70ug of cannflavin per daily dose unit, at least about 80ug of cannflavin per daily dose unit, at least about 90ug of cannflavin per daily dose unit, at least about 100ug of cannflavin per daily dose unit, at least about 110ug of cannflavin per daily dose unit, at least about 120ug of cannflavin per daily dose unit, at least about 130ug of cannflavin per daily dose unit, or at least about 140ug of cannflavin per daily dose unit . Each possibility is a separate embodiment.

According to some embodiments, the predetermined minimal amount of cannflavin comprises between about 50ug and about 120ug of cannflavin per daily dose unit, between about 50ug and about 100ug of cannflavin per daily dose unit, between about 50ug and about 90ug of cannflavin per daily dose unit, between about 60ug and about 120ug of cannflavin per daily dose unit, between about 60ug and about 100ug of cannflavin per daily dose unit, between about 60ug and about 90ug of cannflavin per daily dose unit. Each possibility is a separate embodiment.

The term "**daily dose**" refers to an amount that is consumed per day. In some of the herein disclosed embodiments, it may be understood with respect to the total (i.e., cumulative) amount of cannflavin to be consumed daily in order to obtain an efficient amelioration of pain (e.g., at least 575ug/day or at least 1000ug/day). In some of the herein disclosed embodiments, it may also be understood with respect to the cannabis composition/product referring to the actual total dosage a subject consumes from the cannabis product per day (or to the total dosage that is recommended to the subject to consume per day) in order to ensure that the dosage includes a minimal amount of at least 575ug or at least 800ug CBCA and/or CBC.

For example, a cannabis composition/product prepared, according to the non-limiting standardized extracts of formulas 1-10 (in **Example 9**), contain between 0.1% and 3.0% CBC, that correspond to 1.0mg CBC and 30.0mg CBC per 1g of cannabis product. In some embodiments, a subject that is recommended to consume 0.6g per day of these cannabis products, is administered with a daily amount of between 600ug and 18mg of CBC, respectively.

The terms "daily dose", "per day", "per daily dose unit" may be interchangeably used.

An effective amount of the cannabis composition and the daily dose, necessary to achieve a therapeutic effect, may vary according to factors such as the age, sex, and weight of the subject. Dosage regimens can be adjusted to provide the optimum therapeutic response.

As noted above, the predetermined amount/concentration defines the minimal threshold of CBCA and/or CBC concentrations advantageously and surprisingly found to be essential for efficacious treatment that encompasses a steady therapeutic effect of ameliorating/alleviating pain, and it should not be considered as limiting the content of CBCA and/or CBC in the disclosed compositions and standardized extracts of the invention.

In some embodiment, the cannabis composition comprises a maximal amount/concentration of CBCA and/or CBC of about 1.0mg, per daily dose. Each possibility is a separate embodiment.

In some embodiment, the maximal amount/concentration of CBCA and/or CBC in the cannabis composition/suitable composition/standardized extract is about 1,000,000ug per daily dose, about 750,000ug per daily dose, 500,000ug per daily dose, about 400,000ug per daily dose, about 350,000ug per daily dose, about 300,000ug per daily dose, about 250,000ug per daily dose, about 200,000ug per daily dose, about 150,000ug per daily dose, about 100,000ug per daily dose, about 80,000ug per daily dose, about 60,000ug per daily dose, about 40,000ug per daily dose, about 30,000ug per daily dose, about 20,000ug per daily dose, about 15,000ug per daily dose, about 10,000ug per daily dose, about 7,500ug per daily dose, about 5,000ug per daily dose, about 2,500ug per daily dose, about 1,500ug per daily, about 1,250ug per daily dose, about 1,000ug per daily dose, about 900ug per daily dose, about 800ug per daily dose, about 700ug per daily dose, or about 600ug per daily dose,. Each possibility is a separate embodiment.

In some embodiment, the cannabis composition comprises a content of CBCA and/or CBC between the predetermined minimal amount/concentration and a maximum amount/concentration of about 1.0g, per daily dose. Each possibility is a separate embodiment.

In some embodiment, the cannabis composition comprises a content of CBCA and/or CBC between the predetermined minimal amount/concentration and any one of the maximum amount/concentration of CBCA and/or CBC. Each possibility is a separate embodiment.

As a non-limiting example for the included ranges of CBCA/CBC in the composition, in some embodiments, the cannabis composition may comprise a content of CBCA and/or CBC between the predetermined minimal amount/concentration of any one of 575/800/1000/1300 ug/day, or any other value of minimal amount/concentration between 575ug/day and 2400ug/day. Each possibility is a separate embodiment.

According to some embodiments, the herein disclosed cannabis composition comprises at least a minimal amount/concentration of CBCA/CBC; according to some embodiments, the herein disclosed cannabis composition comprises cannflavin at a concentration of at least 575ug or 800ug per daily dose; according to some embodiments, the herein disclosed cannabis composition comprises at least 1000ug/day or 1300ug/day of CBCA/CBC. Each possibility is a separate embodiment.

In some embodiments, the cannabis composition further comprises cannflavin A at an amount of at least 50ug, per daily dose, or at least 60ug, per daily dose.

According to some embodiments, the cannabis composition is enriched with CBCA and/or CBC. Each possibility is a separate embodiment.

In some embodiments, the cannabis composition enriched with cannflavin A.

As used herein, the term "**enriched**" refers to a cannabis composition and standardized extracts including CBCA and/or CBC or cannflavin at amount/concentration of at least 1.1 times the predetermined minimal amount/concentration of CBCA and/or CBC (e.g., at least 1.1 x575/1.1x800/1.1x1000/1.1x1300 ug/per daily dose), and is limited only by the maximum amount/concentration of no more than about 1.0g per daily dose. Each possibility is a separate embodiment.

In some embodiments, the cannabis composition enriched with CBCA and/or CBC comprises at least 1.1 times, comprises at least 1.2 times, comprises at least about 1.5 times, comprises at least about 2 times, sometimes at least about 4, or at least about 10 times, or sometimes at least about 30 times, or at least about 100 times, sometimes at least about 200 times the predetermined minimal amount/concentration of CBCA and/or CBC, sometimes at least about 500 times the predetermined minimal amount/concentration of CBCA and/or CBC, or sometimes at least about 2000 times the predetermined minimal amount/concentration of CBCA and/or CBC. Each possibility is a separate embodiment.

In some embodiments, the cannabis composition enriched with cannflavin A comprises at least 1.1 times, comprises at least 1.2 times, comprises at least 1.5 times, comprises at least 2 times, sometimes at least 4, or at least 10 times, or sometimes at least about 30 times, or at least about 100 times, sometimes at least about 200 times the predetermined minimal amount/concentration of cannflavin A, sometimes at least about 500 times the predetermined minimal amount/concentration of cannflavin A, or sometimes at least about 2000 times the predetermined minimal amount/concentration of cannflavin A. Each possibility is a separate embodiment.

In some embodiment, the composition comprises CBCA and/or CBC at maximal amount/concentration of less than 20.00% or less than 10.00%. Each possibility is a separate embodiment.

In some embodiment, the composition comprises CBCA and/or CBC at maximal concentration of less than about 20.00%, less than about 15.00%, less than about 10.00%, less than about 9.00%, less than about 8.00%, less than about 7.00%, less than about 6.00%, less than 5.0000%, less than 4.0000%, less than 3.0000%, less than 2.0000%, less than 1.0000%, less than 0.9000%, less than 0.8000%, less than 0.7000%, less than 0.6000%, less than 0.5000%, less than 0.4000%, less than 0.3000%, less than 0.2000%, less than 0.1000%, less than 0.05%, or less than 0.01%, or less. Each possibility is a separate embodiment.

In some embodiment, the maximal concentration of CBCA and/or CBC in the composition is between about 20.00% and about 0.01%, between about 9.0% and about 0.01%, between about 20.00% and about 0.1%, between about 9.0% and about 0.1%, between about 5.00% and about 0.1%, between about 4.00% and about 0.1%, or between about 3.00% and about 0.1%. Each possibility is a separate embodiment.

In some embodiment, the maximal concentration of cannflavin A in the composition is between about 5.00% and about 0.0001%, between about 1.50% and about 0.0001%, between about 1.0% and about 0.005%, or between about 0.5% and about 0.001%. Each possibility is a separate embodiment.

According to some embodiments, the composition contains CBCA/CBC as the only cannabis-derived compound. According to some embodiments, the composition contains CBCA/CBC with THC, and/or CBD as the only cannabinoids. Each possibility is a separate embodiment. According to some embodiments, the composition comprises THC, CBD, and CBCA/CBC. According to some embodiments, the composition comprising CBCA/CBC further comprises cannflavin A. According to some embodiments, the composition comprising CBCA/CBC further comprises at least one additional cannabinoid, for example CBN, CBGA or cannflavin A. Each possibility is a separate embodiment.

According to some embodiments, the disclosed cannabis composition can be further characterized by a minimal ratio (%) of CBCA/CBC and THC concentrations (CBCA and/or CBC / total THC ratio (%)). Each possibility is a separate embodiment.

In some embodiments, the composition comprises CBCA and/or CBC and total THC concentrations characterized by CBCA and/or CBC to total THC ratio of at least about 0.8%. Each possibility is a separate embodiment.

In some embodiments, the composition comprises CBCA and/or CBC and total THC concentrations characterized by CBCA and/or CBC to total THC ratio of at least about 0.8%, at least about 1.00%; or at least about 1.40%. Each possibility is a separate embodiment.

In some specific embodiments, the composition comprises CBCA and/or CBC and total THC concentrations characterized by CBCA and/or CBC to total THC ratio of at least about 0.8% (about 1:120), at least about 1.00% (about 1:100); at least about 1.40% (about 1:71), at least about 5% (about 1:20), at least about 10% (about 1:10), at least about 15% (about 1:7), or at least about 20% (about 1:5). Each possibility is a separate embodiment.

In some related embodiments, the composition comprises CBCA and/or CBC and total THC concentrations characterized by CBCA and/or CBC to total THC ratio between about 0.8% (about 1:120) and about 20% (about 1:5), between about 0.8% (about 1:120) and about 10% (about 1:10), or between about 0.8% (about 1:120) and about 5% (about 1:20). Each possibility is a separate embodiment.

The composition may be further characterized by the content of cannflavin A and total THC. In some embodiments, the composition comprises cannflavin A and total THC concentrations characterized by cannflavin A to total THC of ratio of at least about 0.070%.

In some embodiments, the cannflavin A and total THC concentrations are characterized by a ratio of cannflavin A to total THC of at least about 0.07%, at least about 0.08%, at least about 0.085%, at least about 0.09%, at least about 0.10%, at least about 0.11%, at least about 0.12%, at least about 0.15%, at least about 0.20%, at least about 0.25%, at least about 0.30%, at least about 0.35%, at least about 0.45%, at least about 0.50%, at least about 0.60%, at least about 0.70%, at least about 0.90%, at least about 1.00%, at least about 5.00%, at least about 10.00%, at least about 25.00%, at least about 50.00%, at least about 75.00%, at least about 100.00%, at least about 500.00%, or more. Each possibility is a separate embodiment.

In some embodiments, the cannflavin A and total THC concentrations are characterized by a ratio of cannflavin A to total THC of between about 0.07% and about 1000.00%, a ratio of between about 0.07% and about 500.00%, a ratio of between about 0.07% and about 100.00%, a ratio of between about 0.07% and about 25.00%, between about 0.07% and about 10.00%, between about 0.07% and about 5.00%, or between about 0.07% and about 1.00%, between about 0.07% and about 0.50%, or between about 0.07% and about 0.20%. Each possibility is a separate embodiment.

According to some embodiments, the disclosed cannabis composition may be further characterized by a minimal ratio (%) of CBCA and/or CBC and total CBD concentrations (CBCA and/or CBC / total CBD ratio (%)). Each possibility is a separate embodiment.

In some embodiments, the composition comprises CBCA and/or CBC and total CBD concentrations characterized by CBCA or total CBC to total CBD ratio of at least about 300%. Each possibility is a separate embodiment.

In some embodiments, the composition comprises CBCA and/or CBC and total CBD concentrations characterized by CBCA or total CBC to total CBD ratio of at least about 300%, at least about 330%, at least about 400%; at least about 455%; at least about 475%; or at least about 550%. Each possibility is a separate embodiment.

The composition may be further characterized by the content of cannflavin A and total CBD. In some embodiments, the composition comprises cannflavin A and total CBD concentrations characterized by cannflavin A to total CBD a ratio of at least about 25%.

In some embodiments, the cannflavin A and total CBD concentrations are characterized by a ratio of cannflavin A to total CBD of at least about 25.0%, at least about 30.0%, at least about 50.0%, at least about 75.0%, at least about 100%, at least about 250%, at least 500%, at least about 750%, at least about 1000%, at least about 5000%, at least about 10,000%, at least about 100,000%, or more. Each possibility is a separate embodiment.

In some embodiments, the cannflavin A and total CBD concentrations are characterized by a ratio of cannflavin A to total CBD of between about 25% and about 200,000.0%, a ratio of between about 25% and about 50,000.0%, a ratio of between about 25% and about 20,000.0%, a ratio of between about 25% and about 10,000.0%, a ratio of between about 30% and about 1,000.0%. Each possibility is a separate embodiment.

The composition may be further characterized by the content of CBCA and/or CBC and CBGA. In some embodiments, the composition comprises CBCA and/or CBC at a higher amount/concentration than CBGA/CBG or total CBG (calculated as CBGA*0.877+CBG). Each possibility is a separate embodiment.

In some embodiments, the composition comprises CBCA and/or CBC at amount/concentration of more than about 50% than the amount/concentration of CBGA/CBG. In some embodiments, the composition comprises CBCA and/or CBC at amount/concentration of about 50%, about 75%, about 100%, about 500%, about 1,000%, about 10,000%, about 50,000%, or higher than the amount/concentration of CBGA/CBG. Each possibility is a separate embodiment.

In some embodiments, the composition comprises CBCA and/or CBC at an amount/concentration of between about 50% of the amount/concentration of CBGA/CBG and up to about 100,000% of the amount/concentration of CBGA/CBG. Each possibility is a separate embodiment.

According to some embodiments, the cannabis composition comprises raw cannabis.

According to some embodiments, at least a portion of the CBCA and/or CBC in the composition comprises extracted, isolated, purified, and/or synthetic CBCA and/or CBC, or any combination thereof. Each possibility is a separate embodiment.

According to some embodiments, at least a portion of the CBCA and/or CBC in the composition comprises isolated and/or synthetic CBCA and/or CBC. Each possibility is a separate embodiment.

According to some embodiments, the composition is selected from a plurality of potential cannabis compositions based on an analysis of the content of CBCA and/or CBC in the plurality of potential cannabis compositions. Each possibility is a separate embodiment.

According to some embodiments, CBCA and/or CBC is added to the composition if the CBCA and/or CBC content is below the predetermined minimal amount/concentration. Each possibility is a separate embodiment.

According to some embodiments, at least a portion of the CBCA and/or CBC added to the composition comprises isolated and/or synthetic CBCA and/or CBC. Each possibility is a separate embodiment.

According to some embodiments, the composition is selected based on an analysis/evaluation of the content of CBCA and/or CBC in the plurality of cannabis compositions. According to some embodiments, based on the analysis of the content CBCA and/or CBC is added to the composition to reach the predetermined minimal content, or above. According to some embodiments CBCA and/or CBC is added to the composition if the content of CBCA and/or CBC measured is below a predetermined minimal amount/concentration. Each possibility is a separate embodiment.

According to some embodiments, administration of the cannabis composition comprises administering by route of smoking, inhalation, vaporization, oral administration, buccal administration, nasal administration, topical administration, transdermal administration parenteral administration, or any combination thereof. Each possibility is a separate embodiment.

According to yet another embodiment, a combination of CBCA and/or CBC at the minimum predetermined concentration of at least about 575, at least about 800, at least about 1000, or at least about 1300ug/day and cannflavin A at minimal concentration of at least about 50µg/day, predicts/correlates with treatment efficacy of one or more of the pain-related conditions and/or symptoms, including but not limited to pain of headaches. Each possibility is a separate embodiment.

In some embodiments, the cannabis composition or the standardized extract comprises CBCA and/or CBC at least at its minimal predetermined amount/concentration and additionally cannflavin A at minimal concentration of at least 50µg per daily dose.

According to some embodiments, the cannabis composition comprises cannflavin A at minimal concentrations of at least about 50µg/day. According to some embodiments, the cannabis composition comprises cannflavin A at concentrations of at least about60µg/day.

In some embodiments, the cannabis composition comprises cannflavin A at a minimal concentration of at least about 50µg per daily dose, at least about 60ug per daily dose, at least about 70ug per daily dose, at least about 80ug per daily dose, at least about 90ug per daily dose, at least about 100ug per daily dose, at least about 120ug per daily dose, at least about 140ug per daily dose or more. Each possibility is a separate embodiment.

In some embodiments, the cannabis composition comprises cannflavin A at a maximal concentration of about 100,000ug per daily dose.

In some embodiments, the cannabis composition comprises cannflavin A at an amount between any one of the minimal concentration of at least about 50µg per daily dose, at least about 60ug per daily dose, at least about 70ug per daily dose, at least about 80ug per daily dose, at least about 90ug per daily dose, at least about 100ug per daily dose, at least about 120ug per daily dose, ,at least about 140ug per daily dose and a maximal concentration of about at least about 100,000ug per daily dose. Each possibility is a separate embodiment.

In some embodiments, the minimal amount of cannflavin A includes any dose of cannflavin A (ug/day) determined based on, and corresponding to: at least the 50% percentile, at least the 55% percentile, at least the 60% percentile, at least the 65% percentile, at least the 70% percentile, at least the 75% percentile, at least the 80% percentile, at least the 85% percentile, at least the 90% percentile, at least the 95% percentile, at least the 99% percentile, at least the 100% percentile, or more. Each possibility is a separate embodiment.

According to some embodiments, the cannabis composition comprises cannflavin A at an amount of at least about 50ug/day for use in treating one or more pain-associated conditions and/or symptoms, selected from orthopedic pain, neuropathic pain, spinal disc herniation pain, fibromyalgia (FM) pain, headaches and/or migraines, skeleto-muscle pain, widespread pain, and/or abdominal pain, or any combination thereof, but most particularly pain of headaches. Each possibility is a separate embodiment.

According to some embodiments, the cannabis composition comprises total cannflavin A at an amount of at least about 50ug for use in treating pain, preferably pain of headaches.

In some embodiments, the pain includes, but is not limited to, chronic and/or acute pain of one or more of: orthopedic pain, neuropathic pain, spinal disc herniation pain, fibromyalgia (FM) pain, widespread pain, muscles pain, headaches, and/or abdominal pain, or any combination thereof. Each possibility is a separate embodiment.

According to some embodiments, cannflavin A is added to the cannabis composition or the standardized extract. According to some embodiments, cannflavin A is added to the cannabis composition or the standardized extract at a minimal amount of at least 50ug/day or at least 60ug/day. Each possibility is a separate embodiment. According to some embodiments, cannflavin A is added to the cannabis composition or the standardized extract when cannflavin A amounts are below a minimal concentration of at least 50ug/day.

The composition comprises extracted, isolated, purified, and/or synthetic cannflavin A, or any combination thereof. Each possibility is a separate embodiment.

According to some embodiments, at least a portion of the cannflavin A added to the composition comprises isolated and/or synthetic cannflavin A. Each possibility is a separate embodiment.

In some additional embodiments, the composition may also be characterized by a minimal CBCA and/or CBC to cannflavin A ratio considered as minimum ratio needed for the efficacious treatment.

Reference is now made to **Example 6.**

In some embodiments thereof, the one or more type(s) of pain or pain-related condition and/or symptom is selected from orthopedic pain, neuropathic pain, spinal disc herniation pain, fibromyalgia (FM) pain, pain of headaches, or any combination thereof. Each possibility is a separate embodiment.

In some embodiments, the one or more type(s) of pain or pain-related condition and/or symptom includes orthopedic pain, neuropathic pain, spinal disc herniation pain, fibromyalgia (FM) pain, and headaches, or any combination thereof. Each possibility is a separate embodiment.

In some embodiments, the one or more type(s) of pain or pain-related condition and/or symptom includes orthopedic pain, neuropathic pain, spinal disc herniation pain, fibromyalgia (FM) pain, or any combination thereof. Each possibility is a separate embodiment.

In some embodiments, the one or more type(s) of pain or pain-related condition and/or symptom includes orthopedic pain, neuropathic pain, and spinal disc herniation pain, or any combination thereof. Each possibility is a separate embodiment.

In some embodiments, the one or more type(s) of pain or pain-related condition and/or symptom includes fibromyalgia (FM) pain.

In some embodiments, the one or more type(s) of pain or pain-related condition and/or symptom includes headaches.

According to some embodiments, at least one type(s) of pain or pain-related condition and/or symptom is characterized by a high level of perceived severity. In some embodiments, the high level of perceived severity comprises self-reported ranking of 4 or 5 on a scale of 1-5. Each possibility is a separate embodiment.

In some embodiments, the high level of perceived severity comprises self-reported ranking of 5 on a scale of 1-5.

As used herein the term "**severity**" refers to the perceived severity of the condition and/or symptom as were self-reported on a scale of 1-5 by patients in questionnaires, as exemplified throughout the overview of the study and Examples 1-6. Accordingly, a high level of severity includes self-reported ranking of 4 or 5 out of 5.

In some embodiments, the one or more type(s) of pain or pain-related condition and/or symptom is a comorbidity associated with at least one additional medical condition. Each possibility is a separate embodiment.

As used herein the term "**comorbid condition and/or symptom**" or "comorbidity" or refers to one or more of the pain or pain-related condition and/or symptom of the present invention including when these simultaneously exist with (i.e., co-exist with or associated with) at least one other/additional medical condition in the same patient (or population of patients), and regardless of their causal relationship (i.e., dependent or independent).

In some embodiments, comorbidity refers to pain or pain-related a condition and/or symptom that is associated with at least one additional medical condition and/or symptom, including but not limited to any one of Orthopedic Pain, Sleep Difficulties, Fatigue, Depressive symptoms, Anxiety ,Neuropathic Pain, Spinal Disc herniation, Decreased/Lack of Appetite, Cognitive difficulties (Concentrating, Brain Fog, memory), Headaches and/or Migraine, Fibromyalgia (FM), Nausea and/or Vomiting, PTSD, RA, and Cancer, or any combination thereof. Each possibility is a separate embodiment.

According to some embodiments, the pain is associated with at least one additional medical condition.

In some embodiments, the at least one additional medical condition is selected from Orthopedic Pain, Sleep Difficulties, Fatigue, Depressive symptoms, Anxiety, Neuropathic Pain, Spinal Disc herniation, Decreased/Lack of Appetite, Cognitive difficulties (Concentrating, Brain Fog, memory), Headaches and/or Migraine , Fibromyalgia (FM), Nausea and/or Vomiting , PTSD, rheumatoid arthritis (RA), and Cancer, or any combination thereof. Each possibility is a separate embodiment.

In some embodiments, pain refers to persistent pain that lasts for an extended period or to acute pain, each separately and combined. Each possibility is a separate embodiment.

In some embodiments, the pain may include chronic pain and/or acute pain. Each possibility is a separate embodiment.

According to some embodiments, the cannabis composition comprises a standardized extract.

As used herein, the term "cannabis extract" refers to an herbal preparation prepared by extraction of cannabis plant material.

According to some embodiments, extraction of a cannabis extract may be achieved by various techniques, including but not limited to using solvents, like alcohols or other organic solvents, gasses like CO₂ and butane, and/or to physical methods like presses and extruders. Each possibility is a different embodiment.

The herein disclosed cannabis composition may comprise such a standardized extract in some embodiments thereof, and in some instances the herein disclosed cannabis composition may refer to such a standardized extract.

In some embodiments, a standardized extract or standardized cannabis extract refer to cannabis extracted from a natural source and modified/formulated/standardized preferably to include (by addition) at least a certain amount/content of one or more active ingredient, such as but not limited to: cannabinoids like CBCA and/or CBC, THC, CBD, or cannflavin A, nevertheless according to less favorable embodiments of the invention the standardized extract may also be formulated substantially without THC and/or CBD (possibly by excluding them or by using cannabis extracts having poor THC and/or CBD content). Each possibility is a separate embodiment.

According to some embodiments, standardization can be achieved by mixing extracts from different sources and active ingredients ratios, with or without dilution with excipients. Each possibility is a separate embodiment.

According to some embodiments, standardization can be achieved by adding to the formulation extracted, isolated, purified, and/or synthetic active ingredient. Each possibility is a separate embodiment.

According to some embodiments, standardization can be achieved by excluding from the extracted formulation an active ingredient.

According to some embodiments, the cannabis extract or standardized extract comprises CBCA and/or CBC. Each possibility is a separate embodiment.

According to some embodiments, the standardized cannabis extract essentially consists of CBCA and/or CBC. Each possibility is a separate embodiment.

According to some embodiments, the cannabis extract or standardized extract comprises THC rich content. Each possibility is a separate embodiment.

According to some embodiments, the cannabis extract or standardized extract comprises CBD rich content. Each possibility is a separate embodiment.

According to some embodiments, the cannabis extract or standardized extract has THC dominance. Each possibility is a separate embodiment.

According to some embodiments, the cannabis extract or standardized extract has CBD dominance. Each possibility is a separate embodiment.

According to some embodiments, the cannabis extract or standardized extract has a balanced THC and CBD content. Each possibility is a separate embodiment.

According to some embodiments, the cannabis extract or standardized extract comprises cannflavin A. Each possibility is a separate embodiment.

As used herein, in some embodiments, a cannabis extract having a THC rich content may refer to cannabis extracts having a THC content greater by at least about 2 times, at least about 20 times, at least about 50 times, at least about 100 times, or at least about 200 times, than its CBD content. Each possibility is a different embodiment.

As used herein, in some embodiments, a cannabis extract having a CBD rich content may refer to cannabis extracts having a CBD content greater by at least about 2 times, at least about 20 times, at least about 50 times, at least about 100 times, at least about 200 times, than its THC content. Each possibility is a different embodiment.

As used herein, in some embodiments, a cannabis extract with THC dominance may have a THC content greater than its CBD content by between about 1 to about 20 times.

As used herein, in some embodiments, a cannabis extract with CBD dominance may have a CBD content greater than its THC content by between about 1 to about 20 times.

As used herein, in some embodiments, a cannabis extract with balanced THC and CBD content may comprise contents of THC and CBD within the range of a ratio of THC:CBD 1:2 to a ratio of THC:CBD 2: 1.

In some embodiments, the cannabis composition or the standardized extract comprises at least about 2.6% (w/w) THC, at least about 2.6% (w/w) CBD, at least about 0.005% (w/w) cannflavin and optionally 0.5% (w/w) Vitamin E. Each possibility is a separate embodiment.

In some embodiments, the cannabis composition or the standardized extract comprises at least about 2.6% (w/w) THC, at least about 2.6% (w/w) CBD, at least about 0.1 % (w/w) CBCA and/or CBC, and optionally 0.5% (w/w) Vitamin E. Each possibility is a separate embodiment.

In some embodiments, the cannabis composition or the standardized extract comprises THC and/or CBD concentrations of at least about 2.5% (w/w) each, at least about 5.0% (w/w) each, at least about 7.5% (w/w) each, at least about 10% (w/w) each, at least about 12.5% (w/w) each, at least about 15% (w/w) each, at least about 17.5% (w/w) each, at least about 20% (w/w) each, at least about 25% (w/w) each, at least about 30% (w/w) each, or at least about 35% (w/w) each, or at least about 40% (w/w) each, or any combination thereof. Each possibility is a separate embodiment.

In some embodiments, the cannabis composition or the standardized extract comprises THC and/or CBD concentrations of between about 2.5% (w/w) and about 50% (w/w) each, between about 5.0% (w/w) and about 40% (w/w) each, between about 7.5% (w/w) and about 35% (w/w) each, between about 10% (w/w) and about 30% (w/w) each, or between about 15% (w/w) and about 25% (w/w) each, or any combination thereof. Each possibility is a separate embodiment.

In some embodiments, the cannabis composition or the standardized extract comprises CBCA and/or CBC concentration between 0.01% (w/w) and 20.00% (w/w).

In some embodiment, the composition comprises CBCA and/or CBC at maximal concentration of up to about 20.00%, up to about 15.00%, up to about 10.00%, up to about 9.00%, up to about 8.00%, up to about 7.00%, up to about 6.00%, up to about 5.0000%, up to about 4.0000%, up to about 3.0000%, up to about 2.0000%, up to about 1.0000%, up to about 0.9000%, up to about 0.8000%, up to about 0.7000%, up to about 0.6000%, up to about 0.5000%, up to about 0.4000%, up to about 0.3000%, up to about 0.2000%, up to about 0.1000%, up to about 0.05%, or up to about 0.01%, or less.

In some embodiment, the maximal concentration of CBCA and/or CBC in the composition is between about 20.00% and about 0.01%, between about 9.0% and about 0.01%, between about 20.00% and about 0.1%, between about 9.0% and about 0.1%, between about 5.00% and about 0.01%, between about 5.00% and about 0.1%, between about 4.00% and about 0.1%, or between about 3.00% and about 0.1%. Each possibility is a separate embodiment.

In some embodiments, the cannabis composition or the standardized extract comprises CBCA and/or CBC concentration of: at least about 0.1% (w/w), at least about 0.17% (w/w), at least about 0.2% (w/w), at least about 0.3% (w/w), at least about 0.34% (w/w), at least about 0.6% (w/w), at least about 2.4% (w/w), or at least about 3.0% (w/w).

In some embodiments, the cannabis composition or the standardized extract includes any one of formulations 1-10. Each possibility is a separate embodiment.

In some embodiments, the cannabis composition or the standardized extract includes CBCA and/or CBC, THC, CBD, and/or cannflavin A, at amount (mg/g)/concentrations (%) according to any one of formulations 1-10. Each possibility is a separate embodiment.

Reference is made to **Example 9.**

According to some embodiments, the cannabis composition comprises cannabis extracts enriched with CBCA and/or CBC. Each possibility is a separate embodiment.

In some embodiments, the enriched standardized cannabis extract, or the cannabis composition comprising enriched cannabis extracts, includes extracted, isolated, purified, or synthetic CBCA and/or CBC. Each possibility is a separate embodiment.

According to some embodiments, the cannabis extract includes enriched levels of CBCA and/or CBC from natural source; According to some embodiments, cannabis extract includes enriched levels of CBCA and/or CBC isolated or purified from a natural source; According to some embodiments, cannabis extract includes enriched levels of synthetic CBCA and/or CBC. Each possibility is a separate embodiment.

In some embodiments, the cannabis extracts enriched with CBCA and/or CBC comprises at least 1.1 times or at least 1.2 times, or at least about 1.5 times, or at least about 2 times, or at least about 4 times, or at least about 10 times, or at least about 20 times, or at least about 100 times, or more, the predetermined minimal amount/concentration of CBCA and/or CBC. Each possibility is a separate embodiment.

In some embodiments, the cannabis extract enriched with CBCA and/or CBC comprises at least about 200 times the predetermined minimal amount/concentration of CBCA and/or CBC. Each possibility is a separate embodiment.

In some embodiments, a standardized extracts is enriched with CBCA and/or CBC; In some embodiments, a standardized extracts enriched with CBCA and/or CBC includes an amount/concentration of at least 1.1 times or 1.2 times, or more, the predetermined minimal amount/concentration of CBCA and/or CBC (e.g., at least 575*1.1 ug per daily dose), and is limited only by the maximum amount/concentration of about 1.0g per daily dose. Each possibility is a separate embodiment.

In some embodiments, the cannabis composition comprises a maximal amount/concentration of CBCA and/or CBC of about 1.0ug, per daily dose.

In some embodiments, the maximal amount/concentration of CBCA and/or CBC in the cannabis composition/suitable composition/standardized extract is about 1,000,000ug per daily dose, 750,000ug per daily dose, 500,000ug per daily dose, about 400,000ug per daily dose, about 350,000ug per daily dose, about 300,000ug per daily dose, about 250,000ug per daily dose, about 200,000ug per daily dose, about 150,000ug per daily dose, about 100,000ug per daily dose, about 80,000ug per daily dose, about 60,000ug per daily dose, about 40,000ug per daily dose, about 30,000ug per daily dose, about 20,000ug per daily dose, about 15,000ug per daily dose, about 10,000ug per daily dose, about 7,500ug per daily dose, about 5,000ug per daily dose, about 2,500ug per daily dose, about 1,500ug per daily, about 1,250ug per daily dose, about 1,000ug per daily dose, about 900ug per daily dose, about 800ug per daily dose, about 700ug per daily dose, about 600ug per daily dose. Each possibility is a separate embodiment.

In some embodiments, the cannabis composition comprises a content of cannflavin between the predetermined minimal amount/concentration and a maximum amount/concentration of about 1.0g, per daily dose.

In some embodiments, the cannabis composition or the standardized extract further comprises cannflavin A at concentrations of at least about 0.0010% (w/w).

In some embodiments, the cannabis composition or the standardized extract comprises cannflavin A concentration of: at least about 0.0010% (w/w) at least about 0.0087% (w/w), at least about 0.015% (w/w), at least about 0.0174% (w/w), at least about 0.03% (w/w), at least about 0.27% (w/w), or at least about 1.5% (w/w), or at least about 2.0% (w/w), or at least about 3.0% (w/w) or at least about 4.0% (w/w).

In some embodiments, the cannabis composition or the standardized extract includes CBCA and/or CBC, THC, CBD, and/or cannflavin A, at amount (mg/g)/concentrations (%) according to any one of formulations 1-10. Reference is made to **Example 9.**

In some embodiment, the cannabis composition or the standardized extract comprises cannflavin A at maximal amount/concentration of less than 5.0000%, less than 4.0000%, less than 3.0000%, less than 2.0000%, less than 1.0000%, less than 0.9000%, less than 0.8000%, less than 0.7000%, less than 0.6000%, less than 0.5000%, less than 0.4000%, less than 0.3000%, less than 0.2000%, less than 0.1000%, less than about 0.095%, less than about 0.094%, less than about 0.093%, less than about 0.092%, less than about 0.091%, less than about 0.090%, less than about 0.080%, less than about 0.070%, less than about 0.060%, less than about 0.050%, less than about 0.040%, less than about 0.035%, less than about 0.030%, less than about 0.025%, less than about 0.020%, less than about 0.015%, less than about 0.010%,. Each possibility is a separate embodiment.

In some embodiment, the composition or the standardized extract comprises cannflavin A at maximal amount/concentration of less than 5.0000%.

In some embodiment, the maximal concentration of cannflavin A in the composition is between about 5.0000% and about 0.00001%, between about 5.0000% and about 0.0001%, or between about 0.095% and about 0.0001%. Each possibility is a separate embodiment.

According to some embodiments, there is provided a standardized cannabis extract comprising extracts from a natural source and a predetermined minimal amount/concentration of at least 575ug per daily dose of isolated and/or synthetic CBCA and/or CBC.

According to some embodiments, there is provided a standardized cannabis extract comprising extracts from a natural source and a predetermined minimal amount/concentration of at least 50ug per daily dose of isolated and/or synthetic cannflavin A.

As used herein, the term **"natural source"** refers to a plant source and should be interpreted in its broader sense. For example, a natural source may include a genetically modified, gene edited or transgenic plant.

As used herein, the term **"genetically modified"** refers to a gene edited plant or a transgenic plant, and include both cannabis plants, and other non-cannabis plants, genetically modified to express/include a cannabinoid at significantly higher concentrations relative to a plant which is not genetically modified.

According to some embodiments, the cannabis plant is a genetically modified cannabis plant.

According to some embodiments, the cannabis composition comprises extracts from a natural source and a significantly higher concentrations of CBCA and/or CBC purified or isolated from a genetically modified cannabis plant.

According to some embodiments, the cannabis composition or the standardized extract comprising a cannabinoid extracted, purified or isolated from a genetically modified plant comprises a significantly higher concentrations of said extracted, purified or isolated cannabinoid, relative to a same cannabis composition comprising a cannabinoid extracted, purified or isolated from a plant which is not genetically modified.

In some embodiments, significantly higher concentrations of CBCA and/or CBC extracted, purified or isolated from a genetically modified source includes concentration at the same ranges of cannabis composition enriched with CBCA and/or CBC.

As used herein the terms "enriched" and "significantly higher concentration" may be interchangeably used, and refer to the same ranges of concentration.

According to some embodiments, the cannabis standardized extract comprises a cannabinoid extracted, purified or isolated from a genetically modified plant. Each possibility is a separate embodiment.

According to some embodiments, the cannabis composition comprises a CBCA and/or CBC extracted, purified or isolated from a genetically modified cannabis plant. Each possibility is a separate embodiment.

In some embodiment, at least a portion of the CBCA and/or CBC in the cannabis composition comprises extracted, isolated, purified, and/or synthetic CBCA and/or CBC. Each possibility is a separate embodiment.

According to one aspect, there is provided a standardized cannabis extract comprising extracts from a natural source and isolated and/or synthetic CBCA and/or CBC ; wherein the extract comprises CBCA and/or CBC at a predetermined minimal amount/concentration of at least 575ug per daily dose. Each possibility is a separate embodiment.

According to one aspect, there is provided a standardized cannabis extract comprising extracts from a natural source and a predetermined minimal amount/concentration of at least 575ug per daily dose or 800ug per daily dose of isolated and/or synthetic CBCA and/or CBC.

In some embodiments, the standardized cannabis extract comprises a predetermined minimal amount of at least 1000ug per daily dose or 1300ug per daily dose of CBCA and/or CBC.

In some embodiments, the cannabis composition or the standardized extract comprises CBCA and/or CBC at least at its minimal predetermined amount/concentration and additionally cannflavin A at minimal concentration of at least 50µg per daily dose.

According to some embodiments, the cannabis composition further comprises cannflavin A at minimal concentrations of at least 50µg/day. According to some embodiments, the cannabis composition further comprises cannflavin A at concentrations of at least 60µg/day.

According to some embodiments, the cannabis composition further comprises cannflavin A at an amount of at least about 50ug/day for use in treating pain.

According to some embodiments, the cannabis composition further comprises cannflavin A at an amount of at least about 50ug/day for use in treating one or more pain type(s) or pain-related condition and/or symptom, including orthopedic pain, neuropathic pain, spinal disc herniation pain, fibromyalgia (FM) pain, and/or headaches, or any combination thereof, and preferably including headaches. Each possibility is a separate embodiment.

In some embodiments, the pain includes, but is not limited to, chronic and/or acute pain of one or more of: orthopedic pain, neuropathic pain, spinal disc herniation pain, fibromyalgia (FM) pain, and/or headaches, or any combination thereof. Each possibility is a separate embodiment.

e composition comprises extracted, isolated, purified, and/or synthetic cannflavin A, or any combination thereof. Each possibility is a separate embodiment.

According to some embodiments, at least a portion of the cannflavin A added to the composition comprises isolated and/or synthetic cannflavin A. Each possibility is a separate embodiment.

In some embodiments, the standardized cannabis extract comprises at least about 2.6% (w/w) THC, at least about 2.6% (w/w) CBD, at least about 0.01% (w/w) CBCA and/or CBC and optionally 0.5% (w/w) Vitamin E. Each possibility is a separate embodiment.

In some embodiments, the standardized cannabis extract comprises at least about 5.2% (w/w) THC, at least about 5.2% (w/w) CBD, at least about 0.02% (w/w) CBCA and/or CBC and optionally 0.5% (w/w) Vitamin E.

In some embodiments, the standardized cannabis extract comprises THC and/or CBD concentrations of at least about 2.5% (w/w), at least about 5.0% (w/w), at least about 7.5% (w/w), at least about 10% (w/w), at least about 12.5% (w/w), at least about 15% (w/w), at least about 17.5% (w/w), at least about 20% (w/w), at least about 25% (w/w), at least about 30% (w/w), or at least about 35% (w/w), or at least about 40% (w/w), or any combination thereof. Each possibility is a separate embodiment.

In some embodiments, the standardized cannabis extract comprises THC and/or CBD concentrations of between about 2.5% (w/w) and about 50% (w/w), between about 5.0% (w/w) and about 40% (w/w), between about 7.5% (w/w) and about 35% (w/w), between about 10% (w/w) and about 30% (w/w), or between about 15% (w/w) and about 25% (w/w), or any combination thereof. Each possibility is a separate embodiment.

According to some embodiments, the cannabis extract is enriched with CBCA and/or CBC; and wherein the cannabis extract enriched with CBCA and/or CBC comprises at least 1.1 times or at least 1.2 times, or at least about 1.5 times, or at least about 2 times, or at least about 4 times, or at least about 10 times or at least about 20 times, or at least about 100 times, or more, the predetermined minimal amount/concentration of CBCA and/or CBC.

According to some embodiments, the standardized cannabis extract includes CBCA and/or CBC at an amount between the predetermined minimal amount and a maximum amount of about 500,000ug or 1.0g, per daily dose. Each possibility is a separate embodiment. In some embodiment, the standardized cannabis extract comprises a content of CBCA and/or CBC between the predetermined minimal amount/concentration and a maximum amount/concentration of about 1,000,000ug per daily dose, 750,000ug per daily dose, 500,000ug per daily dose, about 400,000ug per daily dose, about 350,000ug per daily dose, about 300,000ug per daily dose, about 250,000ug per daily dose, about 200,000ug per daily dose, about 150,000ug per daily dose, about 100,000ug per daily dose, about 80,000ug per daily dose, about 60,000ug per daily dose, about 40,000ug per daily dose, about 20,000ug per daily dose, or about 10,000ug per daily dose, about 7,500ug per daily dose, about 5,000ug per daily dose, about 2,500ug per daily dose, about 1,500ug per daily, about 1,250ug per daily dose, about 1,000ug per daily dose, about 900ug per daily dose, about 800ug per daily dose, about 700ug per daily dose, or about 600ug per daily dose. Each possibility is a separate embodiment.

In some embodiment, the standardized cannabis extract comprises a content of CBCA and/or CBC between the predetermined minimal amount/concentration and any one of the maximum amount/concentration of CBCA and/or CBC. Each possibility is a separate embodiment.

In some embodiment, the standardized cannabis extract comprises CBCA and/or CBC at maximal concentration of less than about 20.00%, less than about 15.00%, less than about 10.00%, less than about 9.00%, less than about 8.00%, less than about 7.00%, less than about 6.00%, less than 5.0000%, less than 4.0000%, less than 3.0000%, less than 2.0000%, less than 1.0000%, less than 0.9000%, less than 0.8000%, less than 0.7000%, less than 0.6000%, less than 0.5000%, less than 0.4000%, less than 0.3000%, less than 0.2000%, less than 0.1000%, less than 0.05%, or less than 0.01%, or less. Each possibility is a separate embodiment.

In some embodiment, the maximal concentration of CBCA and/or CBC in the standardized cannabis extract is between about 20.00% and about 0.01%, between about 9.0% and about 0.01%, between about 20.00% and about 0.1%, between about 9.0% and about 0.1%, between about 5.00% and about 0.1%, between about 4.00% and about 0.1%, or between about 3.00% and about 0.1%. Each possibility is a separate embodiment.
In some embodiments, the cannabis composition or the standardized extract comprises a content of at least about 26mg/g (25mg/mL) THC, at least about 26mg/g (25mg/mL) CBD, at least about 3mg/g CBCA/CBC and at least about 0.15 mg/g Cannflavin A. Each possibility is a separate embodiment.

In some embodiments, the cannabis composition or the standardized extract comprises a content of at least about 52mg/g (50mg/mL) THC, at least about 52mg/g (50mg/mL) CBD, at least about 6mg/g CBCA/CBC and at least about 0.3 mg/g Cannflavin A. Each possibility is a separate embodiment.

In some embodiments, the cannabis composition or the standardized extract comprises a content of at least about 26mg/g (25mg/mL) THC, at least about 26mg/g (25mg/mL) CBD and at least about 1.7mg/g CBC. Each possibility is a separate embodiment.

In some embodiments, the cannabis composition or the standardized extract comprises a content of at least about 52mg/g (50mg/mL) THC, at least about 52mg/g (50mg/mL) CBD and at least about 3.4mg/g CBC. Each possibility is a separate embodiment.

In some embodiments, the cannabis composition or the standardized extract comprises a content of at least about 368 mg/g THC, at least about 368 mg/g CBD, 2.7 mg/g at least about Cannflavin A and at least about 24 mg/g CBC. Each possibility is a separate embodiment.

In some embodiments, the cannabis composition or the standardized extract comprises a content of at least about 378 mg/g THC, at least about 378 mg/g CBD, at least about 15 mg/g Cannflavin A and at least about 30 mg/g CBC. Each possibility is a separate embodiment.

In some embodiments, the cannabis composition or the standardized extract comprises a content of at least about 26mg/g (25mg/mL) THC, at least about 26mg/g (25mg/mL) CBD, at least about 1 mg/g CBC. Each possibility is a separate embodiment.

In some embodiments, the cannabis composition or the standardized extract comprises a content of at least about 26mg/g (25mg/mL) THC, at least about 26mg/g (25mg/mL) CBD, at least about 1mg/g CBC and 0.087 mg/g Cannflavin A. Each possibility is a separate embodiment.

In some embodiments, the cannabis composition or the standardized extract comprises a content of at least about 52mg/g (50mg/mL) THC, at least about 52mg/g (50mg/mL) CBD, at least about 2mg/g CBC. Each possibility is a separate embodiment.

In some embodiments, the cannabis composition or the standardized extract comprises a content of at least about 52mg/g (50mg/mL) THC, at least about 52mg/g (50mg/mL) CBD, at least about 2mg/g CBC and at least about 0.174 mg/g Cannflavin A. Each possibility is a separate embodiment.

Reference is now made to **Example 9** that details non-limiting examples of the standardized formulation and ways of obtaining them.

According to some aspects, there is provided a method for classifying cannabis compositions as being suitable for the treatment of pain, the method comprising:
receiving a potential cannabis composition;
evaluating the content of CBCA and/or CBC in the potential cannabis composition;
classifying the composition as being suitable for the treatment of one or more type(s) of pain / pain-related condition and/or symptom when the CBCA and/or CBC content is at or above a predetermined minimal amount/concentration. Each possibility is a separate embodiment.

In some embodiments, the one or more type(s) of pain / pain-related condition and/or symptom is selected from orthopedic pain, neuropathic pain, spinal disk herniation pain, fibromyalgia (FM) pain, and headaches pain, or any combination thereof. Each possibility is a separate embodiment.

Wherein, in some embodiments, the predetermined minimal amount is at least 575ug or 800ug, per daily dose. In some embodiments, the predetermined minimal amount is at least 1000ug or 1300ug, per daily dose.

In some embodiments, the method for classifying cannabis compositions further comprising labeling the potential composition as being suitable for the treatment of the one or more type(s) of pain / pain-related condition and/or symptom when the CBCA and/or CBC content is at or above the predetermined minimal amount/concentration.

In some embodiments, labeling the potential composition as being suitable for the treatment of pain includes labeling the composition as being suitable for the treatment of orthopedic pain, neuropathic pain, spinal disk herniation pain, fibromyalgia (FM) pain, and/or headaches pain, or any combination thereof. Each possibility is a separate embodiment.

In some embodiments, labeling the potential composition as being suitable for the treatment of pain includes labeling the composition as being suitable for the treatment of one or more of orthopedic pain, neuropathic pain, spinal disc herniation pain, fibromyalgia (FM) pain, headaches and/or migraines, skeleto-muscle pain, widespread pain, and/or abdominal pain, or any combination thereof.

In some embodiments, labeling the potential composition as being suitable for the treatment of pain includes labeling the composition as being suitable for the treatment of chronic pain and/or acute pain.

In some embodiments, the method for classifying cannabis compositions further comprising labeling the potential composition as being suitable for the treatment of pain when the CBCA and/or CBC content is at or above the predetermined minimal amount/concentration; and wherein the CBCA and/or CBC and total THC concentrations are characterized by CBCA and/or CBC to total THC ratio of at least about 0.8%.

In some embodiments, the method for classifying cannabis compositions further comprising labeling the potential composition as being suitable for the treatment of pain when the CBCA and/or CBC content is at or above the predetermined minimal amount/concentration; and wherein the CBCA and/or CBC and total CBD concentrations are characterized by CBCA and/or CBC to total THC ratio of at least about 300%.

In some embodiments, the method for classifying cannabis compositions, further comprises evaluating the content of cannflavin A in the potential cannabis composition and classifying the composition as being suitable for the treatment of pain when the cannflavin A content is at or above a predetermined minimal amount/concentration of 50ug, per daily dose.

In some embodiments, the method for classifying cannabis compositions further comprising labeling the potential composition as being suitable for the treatment of pain when the CBCA and/or CBC content is at or above the predetermined minimal amount/concentration; and/or the cannflavin A concentration is at or above a minimal amount/concentration of 50ug per daily dose.

According to an aspect of the disclosure, there is provided a method for classifying cannabis compositions as being suitable for the treatment of pain the method comprising: receiving a potential cannabis composition; evaluating the level of CBCA and/or CBC and/or cannflavin A in the potential cannabis composition; classifying the composition as being suitable for the treatment of pain when the amount/concentration of CBCA and/or CBC and/or cannflavin A is equal to or above a predetermined amount; and wherein the one or more condition and/or symptom is selected from one or more of orthopedic pain, neuropathic pain, spinal disc herniation pain, fibromyalgia (FM) pain, headaches and/or migraines, skeleto-muscle pain, widespread pain, and/or abdominal pain, or any combination thereof. Each possibility is a separate embodiment.

According to some embodiments, the method further comprises labeling the potential composition as being suitable for the treatment of pain when the concentration of CBCA and/or CBC and/or cannflavin A is equal to or above the predetermined amount.

In some embodiments, the method further comprising labeling the potential composition as being suitable for the treatment of pain when the predetermined minimal amount of CBCA and/or CBC is at least about 575ug/day, at least about 600ug/day, at least about 700ug/day, at least about 800ug/day, at least about 900ug/day, at least about 900ug/day, at least about 1000ug/day , at least about 1100ug/day , at least about 1200ug/day , at least about 1300ug/day, at least about 1400ug/day, at least about 1500ug/day, at least about 1600ug/day, at least about 1700ug/day, at least about 1800ug/day or more. Each possibility is a separate embodiment.

In some embodiments, the method further comprising labeling the potential composition as being suitable for the treatment of pain when the predetermined minimal amount of cannflavin A is at least about 50ug/day, at least about 60ug/day, at least about 70ug/day, at least about 80ug/day, at least about 90ug/day, at least about 100ug/day, at least about 120ug/day, at least about 140ug/day, or more. Each possibility is a separate embodiment.
In some embodiments, the method for classifying cannabis compositions, further comprising labeling the potential composition as being suitable for the treatment of headaches, when the CBCA and cannflavin A content is at or above the predetermined minimal amount/concentration.

According to some embodiments, the selection comprises choosing according to a product specification which outlines the concentration of the cannabinoids found in the composition. According to some embodiments, the selection comprises choosing according to a measurement/analysis of a cannabis composition/extract. According to some embodiments, the selection comprises measuring the amount/concentration of the cannabinoid, for example by HPLC.

According to some embodiments, the composition is selected based on an analysis of the content of cannflavin A in the plurality of cannabis compositions. According to some embodiments, based on the analysis of the content cannflavin A is added to the composition to reach the predetermined minimal content, or above. According to some embodiments cannflavin A is added to the composition if the content of cannflavin A measured is below a predetermined minimal amount/concentration.

According to some embodiments, the composition is selected based on an analysis of the content of CBCA and/or CBC in the plurality of cannabis compositions. According to some embodiments, based on the analysis of the content CBCA and/or CBC is added to the composition to reach the predetermined minimal content, or above. According to some embodiments CBCA and/or CBC is added to the composition if the content of cannflavin measured is below a predetermined minimal amount/concentration.

According to some embodiments, the composition is selected based on an analysis of the content of CBCA and/or CBC and/or cannflavin A in the plurality of cannabis compositions. According to some embodiments, based on the analysis of the content CBCA and/or CBC and/or cannflavin A is added to the composition to reach the predetermined minimal content, or above. According to some embodiments CBCA and/or CBC and/or cannflavin A is added to the composition if the content of CBCA and/or CBC and/or cannflavin A measured is below a predetermined minimal amount/concentration.

In some embodiments, the cannabis composition is a suitable cannabis composition; in some embodiments, a suitable cannabis composition comprises a standardized extract; in some embodiments, the suitable cannabis composition comprises a predetermined minimal amount/concentration per day of CBCA and/or CBC ; in some embodiments, the suitable cannabis composition comprises a predetermined minimal amount/concentration per day of at least 575/800/1000/1300ug/day CBCA and/or CBC; in some embodiments, the suitable cannabis composition comprises a predetermined minimal amount/concentration per day of at least 50ug cannflavin A.

According to some embodiments, the cannabis composition comprises a standardized extract. According to some embodiments, a potential/suitable/optional cannabis composition comprises a standardized extract. Each possibility is a separate embodiment.

According to some aspects, there is provided a method for treating pain in a subject in need thereof, the method comprising: administering to the subject a therapeutically effective amount of a cannabis composition comprising a CBCA and/or CBC at amount/concentration of at least 575ug per daily dose.

In some embodiments, the predetermined minimal amount is at least 800ug CBCA and/or CBC A, per daily dose. In some embodiments, the predetermined minimal amount is at least 1000ug CBCA and/or CBC A, per daily dose. In some embodiments, the predetermined minimal amount is at least 1300ug CBCA and/or CBCA, per daily dose.

Wherein in some embodiments the pain includes one or more type(s) of pain selected from orthopedic pain, neuropathic pain, spinal disc herniation pain, fibromyalgia (FM) pain, headaches and/or migraines, skeleto-muscle pain, widespread pain, and/or abdominal pain, or any combination thereof; or wherein in some embodiments the pain includes one or more type(s) of pain selected from orthopedic pain, neuropathic pain, spinal disc herniation pain, fibromyalgia (FM) pain, and/or headaches, or any combination thereof. Each possibility is a separate embodiment.

According to some aspects, there is provided a method for treating pain in a subject in need thereof, the method comprising: administering to the subject a therapeutically effective amount of a cannabis composition comprising a cannflavin A at amount/concentration of at least 50ug per daily dose.

In some embodiments, the predetermined minimal amount is at least 60 ug cannflavin A, per daily dose.

According to some aspects, there is provided a method for treating orthopedic pain, neuropathic pain, spinal disc herniation pain, fibromyalgia (FM) pain, and/or headaches in a subject in need thereof, the method comprises administering to the subject a therapeutically effective amount of a cannabis composition comprising a predetermined minimal amount/concentration of at least 575ug per daily dose CBCA and/or CBC. Each possibility is a separate embodiment.

According to some aspects, there is provided a method for treating orthopedic pain, in a subject in need thereof, the method comprises administering to the subject a therapeutically effective amount of a cannabis composition comprising a predetermined minimal amount/concentration of at least 575ug per daily dose CBCA and/or CBC. Each possibility is a separate embodiment.

According to some aspects, there is provided a method for treating neuropathic pain, in a subject in need thereof, the method comprises administering to the subject a therapeutically effective amount of a cannabis composition comprising a predetermined minimal amount/concentration of at least 575ug per daily dose CBCA and/or CBC. Each possibility is a separate embodiment.

According to some aspects, there is provided a method for treating spinal disc herniation pain, in a subject in need thereof, the method comprises administering to the subject a therapeutically effective amount of a cannabis composition comprising a predetermined minimal amount/concentration of at least 575ug per daily dose CBCA and/or CBC. Each possibility is a separate embodiment.

According to some aspects, there is provided a method for treating fibromyalgia (FM) pain in a subject in need thereof, the method comprises administering to the subject a therapeutically effective amount of a cannabis composition comprising a predetermined minimal amount/concentration of at least 575ug per daily dose CBCA and/or CBC. Each possibility is a separate embodiment.

According to some aspects, there is provided a method for treating headaches in a subject in need thereof, the method comprises administering to the subject a therapeutically effective amount of a cannabis composition comprising a predetermined minimal amount/concentration of at least 1000ug per daily dose CBCA and/or CBC. Each possibility is a separate embodiment.

According to some aspects, there is provided a method for treating headaches in a subject in need thereof, the method comprises administering to the subject a therapeutically effective amount of a cannabis composition comprising a predetermined minimal amount/concentration of at least 1000ug per daily dose CBCA and/or CBC and at least 50ug per daily dose cannflavin A.

According to some aspects, there is provided a method for treating headaches in a subject in need thereof, the method comprises administering to the subject a therapeutically effective amount of a cannabis composition comprising a predetermined minimal amount/concentration of at least 50ug per daily dose cannflavin A.

The following examples are presented in order to more fully illustrate some embodiments of the invention. They should in no way be construed, however, as limiting the broad scope of the invention. One skilled in the art can readily devise many variations and modifications of the principles disclosed herein without departing from the scope of the invention.

### EXAMPLES

### Overview of the study presented in Examples 1-6.

The study described in **Examples 1-6** was approved by the Ethics Committee of Shaare Zedek Medical Center, Jerusalem, Israel (Helsinki reference number 00245-21-SZMC).

Clinical data was collected by phone interviews. Patients were eligible to participate in the study if they were Hebrew speaking and aged above 18 years old.

The subjects/patients have standing medical cannabis licenses prescribed for different medical conditions and consumed medical cannabis in the past 6 months (purchased, at least in part, from Panaxia Ltd.).

477 subjects/patients agreed to complete a questionnaire regarding the perceived efficacy (i.e., self-report) of the cannabis they consume to relieve the pain they experience as a symptom or as a condition. The questionnaire was reviewed by a medical doctor.

In the questionnaire, patients were asked to self-evaluate the level of severity of the specific types of pain they suffer from - which included any of orthopedic pain, neuropathic pain, spinal disk herniation pain, fibromyalgia (FM) pain, and/or headaches pain - on a scale of 1-5, where 1 represents low severity/slightly disturbing and 5 represents very severe/not functioning. Similarly, patients were asked to indicate any other/additional medical condition they have (i.e., comorbidities).

The patients also provided, in the questionnaire, answers regarding the perceived efficacy of the cannabis treatment in reducing the level of their pain, including orthopedic pain, neuropathic pain, spinal disk herniation pain, fibromyalgia (FM) pain, and/or headaches pain, on a scale 1-7, where 1 represents lack of any cannabis effect (i.e., inefficient/ineffective treatment) and 7 represents high efficacy of treatment (i.e., extremely effective).

The efficacies reported by each subject were assigned to the medical cannabis composition administered/consumed.

In addition, after the clinical data was collected from the questionnaire and processed, the medical cannabis strains indicated by patients in the questionaries were collected from various approved cultivators in Israel and were chemically analyzed for data about the phytocannabinoid cannabis molecule content of each strain utilizing high-performance liquid chromatography (HPLC) with an Ultraviolet-visible photodiode array (PDA) detector.

Importantly, the chemical analyses were performed on fresh and unopened samples of inflorescence strains including from Panaxia's pharmacy warehouse rather than from samples obtained from the patients.

Using an automated clustering algorithm augmented with manual refinement by analytical chemists, nineteen (19) mutually exclusive cannabis molecules were identified and quantified, by both their concentration relative to THC, retention times (RRT) and PDA UV-spectra.

Combining the clinical and chemical data, an exploratory approach was used to identify which, if any, cannabis molecules were positively correlated with treatment efficacy of any of their various pain-related conditions and/or symptoms, including orthopedic pain, neuropathic pain, spinal disk herniation pain, fibromyalgia (FM) pain, and/or headaches pain.

Using a dedicated software (written in python), the clinical and chemical data were organized, and Spearman correlations were performed for each of these 19 cannabis molecules, between its median concentrations for each strain and the mean efficacy scoring for each strain for treating pain. Multiple testing corrections were done to control for a false discovery rate.

According to this approach a molecule showing a statistically significant high correlation indicates that as its dosage increases the treatment of pain is more effective.

Advantageously, one of the molecular clusters was identified as CBCA based on the following:
1. The peaks comprising this cluster have a RRT range of 1.26-1.28, whereas the RRT of the CBCA standard (provided by Cerilliant, item no. C-150, batch no. FE05091703) is 1.28 (CBCA RT is 17.329, THC RT is 13.532) in the specific HPLC method applied in this study **(****FIG. 1****).**
2. The UV spectra of these peaks match the spectrum of the CBCA standard (compare **FIG. 2A** with **FIG. 2B****).**

Next, spearman correlations were performed between linked chemical and clinical data for each of the pain specific conditions and/or symptoms - specifically, the median concentrations of CBCA and/or total CBC for each strain was correlated to the mean efficacy scoring across each strain for treating of each of the specific pain condition and/or symptom, whereas concentration of total CBC is calculated as (CBCA*0.877 + CBC). Multiple testing corrections were done to control for a false discovery rate.

Advantageously, this analysis showcased a statistically significant high correlation demonstrating that as the dosage of CBCA and/or total CBC increases, the treatment of pain including orthopedic pain, neuropathic pain, spinal disk herniation pain, fibromyalgia pain, and headaches pain, is more effective.

**Overview of the results of Examples 1-6** - as presented in **Table 1** below, and exemplified throughout **Examples 1-6** the above-described approach advantageously and surprisingly identified CBCA/CBC as the molecule most positively correlated (relative to other identified molecules), in a statistically significant manner, with treatment efficacy of several types of pain, namely: (i) orthopedic pain; (ii) neuropathic pain; (iii) spinal disk herniation pain; (iv) fibromyalgia (FM) pain; and (v) headaches pain.

**Table 1: pain associated conditions/symptoms found to highly correlate, in a statistically significant manner, with concentrations of CBCA/total CBC**

| **condition and/or symptom** | **molecule** | **Predetermined minimal amount (ug per daily dose) 50;75 percentiles** | **spearman coefficient (r)** | **p-value** | **FDR corrected p-value** |
|---|---|---|---|---|---|
| orthopedic pain (example 1) | CBCA | 575; 800 | 0.87 | 1.0E-05 | 6.0E-05 |
| neuropathic pain (example 2) | CBCA | 575; 800 | 0.84 | 3.0E-04 | 2.7E-03 |
| spinal disk herniation pain (example 3) | CBCA | 575; 800 | 0.90 | 9.0E-04 | 1.2E-02 |
| pain in population of FM patients (example 4) | CBCA and CBC (total CBC) | 575; 800 | 0.89 | 2.9E-03 | 2.5E-02 |
| Headaches (example 5) | CBCA and CBC (total CBC) | 1000; 1300 | 0.81 | 4.3E-03 | 2.9E-02 |
| Headaches (example 6) | CBCA with cannflavin A | 1000; 1300 with 50; 60 | 0.85 | 6.0E-04 | 1E-3 |

As each strain was assigned with its median CBCA or total CBC amount (% concentration (w/w) of a molecule in the dry cannabis plant sample), the 50% (median) and 75% percentiles were calculated based on all the strains medians.

In accordance with **Table 1,** in some embodiments related to orthopedic pain, neuropathic pain, spinal disk herniation pain and fibromyalgia pain a minimal dose of 575 ug/day of CBCA or total CBC was determined based on the 50% percentile, which corresponds to 0.17% (w/w), and considering minimal consumption of at least about 0.333 grams of cannabis per day by a subject (i.e., 17 milligrams of CBCA per month); and a minimal amount/concentration of 800ug/day of CBCA or total CBC was determined based on the 75% percentile, which corresponds to 0.24% (w/w), and considering minimal consumption of at least about 0.333 grams of cannabis per day by a subject (i.e., 24 milligrams of CBCA per month).

In addition, in some embodiments related to pain of headaches a minimal daily dose of 1000 ug/day CBCA (with cannflavin A) or total CBC was determined based on the batches concentrations 50% percentile, which was 0.3%, and considering minimal daily consumption of at least about 0.333 grams of cannabis inflorescence by a subject (i.e., 10g per month); and a minimal daily dose of 1300ug/day CBCA (with cannflavin A) or total CBC was determined based on the 75% percentile which was 0.4% and considering minimal daily consumption of at least about 0.333 grams by a subject (i.e., 10g per month) **(Table 1)**.

In addition, for each pain-associated condition and/or symptom in each one of **Examples 1-6** below, the median of the ratio of the concentrations of CBCA or total CBC and total THC and that of CBCA or total CBC and total CBD (i.e. CBCA or total CBC divided by total THC or total CBD) across the strains was calculated and considered as the minimum ratio required for efficacious treatment of the condition and/or symptom.

To conclude, the surprising findings disclosed herein, which are the result of a comprehensive clinical and chemical analyses performed on a wide range of data, allow choosing a suitable cannabis composition that fulfills a requirement for a predetermined minimal amount of at least about 575ug or 1000ug of CBCA or total CBC per daily dose, from a plurality of potential/optional compositions, as well as producing cannabis compositions including the same requirement (e.g., standardized extracts).

Advantageously, this ensures a consistent therapeutic effect on patients, making it suitable for treatment of any one of orthopedic pain, neuropathic pain, spinal disk herniation pain, fibromyalgia pain, and/or headaches, or any combination thereof.

### Methods of Examples 1-6 -

Chemical analysis procedure - Samples were prepared by extraction of 7.5 g of cannabis inflorescences with 130 g of methanol using a Distek Prep Engine2 system for 2 min at 5000 rpm. A portion of the prepared solution was collected and filtered through a 0.22 µm PTFE syringe filter. This solution, and a second solution further diluted 1:5, were injected to the HPLC and analyzed for characterization and quantification. The cannabis administered was evaluated by HPLC to identify and quantify its molecular profile (cannabinoids, including CBC and CBCA, and other cannabis molecules, including cannflavin A). The evaluation is based on the fact that different cannabis molecules interact differently with the adsorbent resin included in the HPLC's column, causing different flow rates and leading to the separation in time of the molecules, as they flow out of the column (retention time (RT)).

Computational tools - the following software tools were used: Python (V. 3.8.6) with Pandas, Numpy, Scikit-learn and SciPy packages for statistical analysis; chemical data was stored at PostgreSQL database (V.13.9); DBeaver software (V. 7.3.5) was used for querying chemical data.

### Example 1- Efficacy evaluation of potential cannabis compositions in treating orthopedic pain, and identification of CBCA as a cannabis molecule whose concentration correlates with a positive outcome.

477 subjects/patients, aged above 18 years old with a standing medical cannabis licenses prescribed for different medical conditions and consuming medical cannabis in the past 6 months, agreed to complete a questionnaire regarding the perceived efficacy of the cannabis consumed which was reviewed by a medical doctor. Patients were asked to self-evaluate the level of severity of their pain on a scale of 1-5, where 1 represents low severity/slightly disturbing and 5 represents very severe/not functioning.

Out of these 477 patients, chemical data matching the medical cannabis strains they consumed was located for 323 and analyzed.

Out of these 323 patients, 80 patients for whom there was matching clinical and chemical data according to study protocol guidelines indicated that they were suffering from orthopedic pain (24.7%), 56 subjects of whom suffered from severe pain (70.0%) (Ranked 5 out of 5).

The most common co-morbid self-reported indications of these patients are reported in **Table 3**, indicating their medical background.

**Table 3: Co-morbid condition of the 56 patients having orthopedic pain with level of severity ranked 5.**

| **Medical Background patients with Orthopedic Pain** | |
|---|---|
| **Co-Morbid condition (self-reported)** | **Number of patients (%)** |
| Orthopedic Pain | 56 (100%) |
| Sleep Difficulties | 43 (76%) |
| Fatigue | 36 (64%) |
| Depressive symptoms | 35 (62%) |
| Anxiety | 29 (52%) |
| Neuropathic Pain | 28 (50%) |
| Spinal Disc herniation | 27 (48%) |
| Decreased/Lack of Appetite | 25 (44.6%) |
| Cognitive difficulties (Concentrating, Brain Fog, memory) | 20 (36%) |
| Headaches and/or Migraine | 18 (32%) |
| Fibromyalgia (FM) | 15 (27%) |
| Nausea and/or Vomiting | 8 (14%) |
| PTSD | 8 (14%) |
| RA | 6 (11%) |
| Cancer | 4 (7%) |

The questionnaires included demographic data, such as age, gender, employment status, and years of cannabis, as summarized in **Table 4.**

Patients had a median age of 58 y/o and 33 (59%) of them were males. During the study period, 34% of the patients were employed, while 41% of the patients were unemployed due to their illness. More than 62% of the patients reported that they had used medical cannabis treatment for over 5 years.

**Table 4 - Descriptive Demographic Characteristics of the 56 patients included in this study.**

| **Gender** | **Number of patients, (%)** |
|---|---|
| Male | 33 (59%) |
| Female | 23 (41%) |

| **Age (Years), median= 58, mean= 57.5** | |
|---|---|
| 18-29 | 2 (3.6%) |
| 30-49 | 10 (17.9%) |
| 50-64 | 29 (51.8%) |
| 65+ | 15 (26.8%) |

| **Employment** | |
|---|---|
| Full Time | 9 (16%) |
| Part Time | 9 (16%) |
| Business owner | 1 (1.8%) |
| Unemployed - Personal Reasons | 4 (7.1%) |
| Unemployed - Illness | 23 (41%) |
| Student | 0 (0%) |
| Pensioner | 10 (17.9%) |

| **Medical Cannabis Use Time (Years), median = 6, mean = 8.52** | |
|---|---|
| <1 | 0 (0%) |
| 1 | 1 (1%) |
| 2 | 6 (10.7%) |
| 3 -4 | 8 (14.3%) |
| 5-10 | 25 (44.6%) |
| > 10 | 10 (17.9%) |
| Unknown | 6 (10.7%) |

| **Personal status** | |
|---|---|
| Divorced | 13 (23.2%) |
| Married | 28 (50%) |
| Single | 6 (10.7%) |
| Widowed | 1 (1.8%) |
| Unknown | 8 (14.3%) |

| **Education (in years)** | |
|---|---|
| 0-12 years | 32 (57.1%) |
| 12+ years | 15 (26.8%) |
| unknown | 9 (16.1%) |

| **Country** | |
|---|---|
| Israel | 39 (69.6%) |
| Other | 10 (17.9%) |
| unknown | 7 (12.5%) |

The patients also reported the name of the cannabis strain they consumed, dosage and consumption method (inflorescence smoking or orally consumed oil; only inflorescence products consumed by vaping or smoking were included in the study as there were not enough patient rankings data for oils, and as the merging of chemical and clinical data from oil and inflorescence batches may lead to methodological errors).

The patients reported they administer/consume the cannabis by vaping or smoking any of 17 strains of cannabis, namely Persian Kush, Tel Aviv, London, Wind, Marmalade, Sensi Star, Flame, Tsunami, Ghost Train Haze, Jack Haze, Argaman, Mango Mint, Opal, Afghani, Tripoli, Jean Guy, and Sour Haze.

Importantly, the patients answered a questionnaire regarding the perceived efficacy of the cannabis treatment in reducing the level of pain on a scale of 1-7, where 1 represents lack of any cannabis effect (i.e., ineffective treatment) and 7 represents high efficacy of treatment (i.e., extremely effective).

Only the efficacy scores, belonging to the 56 patients suffering from severe orthopedic pain (i.e. ranked 5 out of 5), were included in this analysis and were correlated with the chemical data.

The efficacies reported by each subject were assigned to the strain administered.

Each strain was assigned a mean effectiveness score which was calculated by averaging the treatment efficacy scores reported by the patients included in this study who administered this strain, and ranked its treatment efficacy for orthopedic pain as severe. These averages, one for each strain, were order-ranked among themselves.

### Identifying cannabinoids correlated with a positive outcome.

A total of 166 inflorescence batches of the cannabis strains reported to be administered were chemically analyzed by HPLC to identify and quantify their molecular profiles (cannabinoids and other cannabis molecules). The evaluation/analysis was based on the fact that different cannabinoids interact differently with the adsorbent resin included in the HPLC's column, causing different flow rates and leading to the separation in time of the cannabinoids, as they flow out of the column.

Peaks of the resulting chromatograms are assumed to be cannabis molecules. Nineteen (19) peaks could be identified based on their UV spectra and relative (to THC) RT (RRT), and as compared with commercial analytical standards. Quantification of each of these cannabis molecules was also performed, using their predetermined relative response factors (RRF) to an ibuprofen external standard.

It is important to note that the chemical analysis by HPLC was performed on inflorescence strains, which typically contain cannabinoids in their acidic form. However, in common consumption methods of inflorescence, patients consume the cannabinoids in their neutral form as they are ingested after being heated above their decarboxylation temperatures, so when inhaled the molecules are already in their neutral form. Thus, for both inflorescence and decarboxylated products (e.g. orally-consumed oils), the neutral forms are considered to have the healing effect *in vivo.* While this research identifies cannabinoids in their acidic form in the inflorescence, it is important to note that the clinical applications are relevant to the neutral forms in decarboxylated products.

The RRT and spectra data for all observed molecules (peaks in the HPLC chromatograms) in all samples were subsequently analyzed by a K-means classification algorithm.

For an initial clustering, a two-part K-means algorithm was applied for clustering molecules, characterized by their RRT, from all the HPLC runs of all the batches. This is an unsupervised machine learning algorithm, used for grouping data into clusters with similar characteristics. Each peak in each HPLC run represents a different, unique molecule, and therefore a constrained version of the K-means algorithm was applied. The constraints were set such that 19 known cannabis molecules will host unique clusters. The number of clusters was automatically determined by Elbow and Silhouette techniques, and each cluster represented a different molecule. This two-part algorithm was first run on the spectra data, grouping all peaks with similar spectra. Then a clustering based on RRT was applied, thus dividing all molecules into clusters using a two-dimensional method, based on both spectra and RRT.

Finally, the resulting clusters were further explored by plotting for each cluster an overlay of the spectra of all its peaks (from the different HPLC runs), allowing easy identification of outlier spectra which most probably belong to another cluster with a nearby RRT. The clusters were refined manually by analytical chemists to ensure that each cluster indeed contained all the peaks, from all HPLC runs, and only these peaks, related to this cluster/molecule.

The concentration of each molecule was evaluated according to the area under the curve value in each chromatogram standardized to the area of an ibuprofen standard injected in each HPLC run. The concentration of each cluster (representing a cannabis molecule) for each of the strains was calculated as the median of the strain's batches. For clusters representing known cannabinoids, absolute amounts were also calculated using their analytical standards.

Advantageously one of the molecular clusters was identified as CBCA based on the following:
1. The peaks comprising this cluster have a RRT range of 1.26-1.28 whereas the RRT of the CBCA standard (provided by Cerilliant, item no. C-150, batch no. FE05091703) is 1.28 (CBCA RT is 17.329, THC RT is 13.532) in the specific HPLC method applied in this study **(****FIG. 1****).**
2. The UV spectra of these peaks match the spectrum of the CBCA standard (compare **FIG. 2A** with **FIG. 2B****).**

The above comprises the chemical data.

### Correlation between CBCA/total CBC concentration and clinical efficacy across strains

To evaluate the potential efficacy of the different cannabis molecules in the treatment of pain, the clinical data and chemical data were statistically analyzed together.

An algorithm was developed which allows identifying peaks recurring in different batches, based on their RRT's and UV spectra, thereby identifying cannabinoids characteristic to certain cannabis strains and for which a correlation was found with efficient or inefficient treatment response as reported by the subjects in the questionnaire.

In short, each cannabis strain was assigned a representative efficacy score, based on the mean efficacy assigned to the strain in the questionnaire. As the efficacy score was provided to a strain, regardless of batch, the chemical data for each strain was taken as the median for each peak (representing a cannabis molecule) across all batches of the strain.

A potential efficacious cannabis molecule's concentration (as measured chemically) should be positively correlated with treatment efficacy (as reported by the patients), across all strains, in a statistically significant manner.

Advantageously, using this approach a molecule showing a statistically significant high correlation indicates that as its dosage increases the treatment of orthopedic pain is more effective.

A Spearman correlation was then used to quantify the correlation between the questionnaire's efficacy of each strain (the label) and the concentration of a specific cannabis molecule in each strain (i.e., the clinical data vs. the chemical data of each of the 19 clusters). The *p*-values were adjusted using the False Discovery Rate (FDR) method, taking into account multiple tests.

Advantageously and surprisingly, it was found that the ranked median concentrations of the cluster representing CBCA across the 17 strains were positively correlated in a statistically significant manner with the order-ranked strains' efficacy scores provided by patients with severe orthopedic pain (i.e., ranked level 5 out of 5).

The Spearman correlation coefficient (r) was 0.87 with a *p*-value of 6.0E-05 after multiple testing corrections using the FDR method, indicating a statistically significant positive correlation between CBCA concentration and treatment efficacy of severe orthopedic pain, as can be seen in **FIG. 3A** and summarized in herein above **Table 1.**

No correlation with efficacy was found for THCA (r=0.346, FDR corrected *p-*value=0.279, original *p*-value=0.097), THC (r=0.016, FDR corrected *p*-value= 0.957, original *p*-value=0.942). Similarly, no correlation with efficacy was found for CBDA (r=0.519, FDR corrected *p*-value=0.06, original *p*-value=0.009), CBD (r=0.38, FDR corrected *p*-value=0.279, original *p*-value=0.132).

Interestingly, and counterposed to the varying literature highlighting dose dependent effects of THC and CBD, neither of their acid (THCA or CBDA) or neutral (THC or CBD) forms were found to be significantly correlated with the effective treatment of orthopedic pain.

As each strain was assigned with its median CBCA amount (% concentration (w/w) of a molecule in the dry cannabis plant sample) from all the strain's batches, the 50% (median) and 75% percentiles were calculated based on all the strains medians.

In accordance, a minimal daily dose of 575ug/day was determined based on the strains concentrations 50% percentile, which was 0.17%, and considering minimal daily consumption of at least about 0.333 grams of cannabis inflorescence by a subject (i.e., 10g per month); and a minimal daily dose of 800ug/day was determined based on the 75% percentile which was 0.24% and considering minimal daily consumption of at least about 0.333 grams by a subject (i.e., 10g per month).

In addition, the CBCA to total THC ratio (calculated as (% CBCA / % total THC) X 100%) and CBCA to total CBD ratio (calculated as (% CBCA / % total CBD) X 100%) was calculated based on their concentrations for each of the 17 strains, whereas total THC is calculated as (THCA*0.877 + THC), and total CBD is calculated as (CBDA*0.877 + CBD).

The median of this ratio 1.05% (rounded to 1.0%) for CBCA to total THC and 398% (rounded to 400%) for CBCA to total CBD can be considered a minimum ratio of CBCA to total THC and/or total CBD, respectively, needed for the efficacious treatment of orthopedic pain.

In conclusion, it was advantageously found that the concentrations of the cluster representing CBCA across the strains were positively correlated, in a statistically significant manner, with the efficacies of these strains in treating/attenuating/reducing/preventing severe orthopedic pain, self-reported by the subjects as level 5 out of 5 i.e., severe pain.

### Example 2 - Efficacy evaluation of potential cannabis compositions in the treatment of neuropathic pain, and identification of CBCA as a cannabis molecule whose concentration correlates with a positive outcome

To evaluate the potential efficacy of the different cannabis molecules in the treatment of additional pain specific conditions and/or symptoms the chemical and clinical data was further analyzed by applying the same general working frame, workflow, and methods described in the **"Overview of the study presented in Examples 1-6"** and in **Example 1.**

The analysis exemplified herein refers to 30 subjects with neuropathic pain who self-evaluated their pain specific condition and/or symptom on a scale of 1-5 and ranked it as 5 out of 5 - i.e., severe pain, and were asked to self-evaluate the level of perceived efficacy of their cannabis treatment in reducing the level of their pain on a scale of 1-7, where 1 represents lack of any cannabis effect (i.e., ineffective treatment) and 7 represents high efficacy of treatment (i.e., extremely effective).

The patients reported they administer/consume the cannabis by vaping or smoking any of 13 strains of cannabis, namely Persian Kush, Tel Aviv, London, Wind, Marmalade, Sensi Star, Flame, Tsunami, Ghost Train Haze, Dark Helmet, Jack Haze, Mango Mint, and Opal.

A potential efficacious cannabis molecule's concentration (as measured chemically) should be positively correlated with treatment efficacy (as reported by the patients), across all strains, in a statistically significant manner. Advantageously, using this approach a molecule showing a statistically significant high correlation indicates that as its dosage increases, the treatment of neuropathic pain is more effective.

A Spearman correlation was then used to quantify the correlation between the questionnaire's efficacy scores of each strain (the label) and the concentration of a specific cannabis molecule in each strain (i.e., the clinical data vs. the chemical data of each of the 19 clusters representing known cannabinoids, including CBCA). Since 19 clusters were tested, the *p*-values were adjusted using the False Discovery Rate (FDR) method, considering multiple tests.

Advantageously and surprisingly, it was found that the concentrations of the cluster representing CBCA (reference is made to **FIG. 1** and **FIG. 2A****-2B** for the method of identification) across 13 strains were positively correlated with the perceived efficacies of these strains in treating/attenuating/preventing neuropathic pain as was self-reported by the subjects.

A Spearman correlation of r=0.84 (*p*-value=2.7E-03, after FDR correction) was found, indicating a statistically significant positive correlation between CBCA concentration and treatment efficacy of neuropathic pain, as can be seen in **FIG. 3B** and summarized in herein above **Table** 1 herein above.

No correlation with efficacy was found for THCA (r=0.302, FDR corrected *p-*value=0.809, original p-value=0.161), THC (r=0.008, FDR corrected *p*-value=0.98, original*p-*value=0.97) or Total THC (r=0.236, FDR corrected *p*-value=0.960, original *p*-value=0.276). Similarly, no correlation with efficacy was found for CBDA (r=0.158, FDR corrected *p-*value=0.976, original *p*-value=0.471), CBD (r=-0.116, FDR corrected *p*-value=0.976, original *p*-value= 0.645) or Total CBD (r=0.005, FDR corrected *p*-value=0.98, original *p*-value=0.98).

Interestingly, and counterposed with the varying literature highlighting dose dependent effects of THC and CBD, neither of their acid forms (THCA or CBDA), neutral forms (THC or CBD) and total values taken together (total THC or total CBD) were found to be significantly correlated with the effective treatment of neuropathic pain.

As each strain was assigned with its median CBCA amount (% concentration (w/w) of a molecule in the dry cannabis plant sample) from all the strain's batches, the 50% (median) and 75% percentiles were calculated based on all the strains medians.

In accordance, a minimal daily dose of 575ug/day was determined based on the strains concentrations 50% percentile, which was 0.17%, and considering minimal daily consumption of at least about 0.333 grams of cannabis inflorescence by a subject (i.e., 10g per month); and a minimal daily dose of 800ug/day was determined based on the 75% percentile which was 0.24% and considering minimal daily consumption of at least about 0.333 grams by a subject (i.e., 10g per month).

In addition, CBCA to total THC ratio (calculated as (% CBCA / % total THC) X 100%) and CBCA to total CBD ratio (calculated as (% CBCA / % total CBD) X 100%) was calculated based on their concentrations for each of the 13 strains. The median of this ratio 1.05% (rounded to 1.0%) for total THC and 455% for total CBD can be considered a minimum ratio of CBCA to total THC and total CBD, respectively, needed for the efficacious treatment of neuropathic pain.

In conclusion, it was advantageously found that the concentrations of the cluster representing CBCA across the strains were positively correlated, in a statistically significant manner, with the efficacies of these strains in treating/attenuating/reducing/preventing severe neuropathic pain self-reported by the subjects as level 5 between 1 to 5, i.e., severe pain.

### Example 3 - Efficacy evaluation of potential cannabis compositions in the treatment of spinal disk herniation pain, and identification of CBCA as a cannabis molecule whose concentration correlates with a positive outcome

To evaluate the potential efficacy of the different cannabis molecules in the treatment of additional pain specific conditions and/or symptoms, the clinical and chemical data was further analyzed, by applying the same general working frame, workflow and methods described in the **"Overview of the study presented in Examples 1-6"** and in **Example 1.**

The analysis exemplified herein refers to 24 subjects with spinal disk herniation pain as main indication who self-evaluated their pain specific condition and/or symptom on a scale of 1-5 and ranked it as 5 out of 5 - i.e., severe pain, and were asked to self-evaluate the level of perceived efficacy of their cannabis treatment in reducing the level of their pain on a scale of 1-7, where 1 represents lack of any cannabis effect (i.e., inefficient/ineffective treatment) and 7 represents high efficacy of treatment (i.e., extremely effective).

The patients reported they administer/consume the cannabis by vaping or smoking any of 9 strains of cannabis, namely Tel Aviv, Marmalade, Sensi Star, Tsunami, Ghost Train Haze, Jack Haze, Tripoli, Sour Haze, Persian Kush.A potential efficacious cannabis molecule's concentration (as measured chemically) should be positively correlated with treatment efficacy (as reported by the patients), across all strains, in a statistically significant manner. Advantageously, using this approach a molecule showing a statistically significant high correlation indicates that as its dosage increases, the treatment of spinal disk herniation pain is more effective.

A Spearman correlation was then used to quantify the correlation between the questionnaire's efficacy of each strain (the label) and the concentration of a specific cannabis molecule in each strain (i.e., the clinical data vs. the chemical data of each of the 19 clusters representing known cannabinoids, including CBCA). Since 19 clusters were tested, the *p-*values were adjusted using the False Discovery Rate (FDR) method, taking into account multiple tests.

Advantageously and surprisingly, it was found that the concentrations of the cluster representing CBCA (reference is made to **FIG. 1** and **FIG. 2A****-2B** for the method of identification) across 9 strains were positively correlated with the perceived efficacies of these strains in treating/attenuating/preventing spinal disk herniation pain as was self-reported by the subjects.

A Spearman correlation of r=0.90 (p-value=1.2E-02, after FDR correction) was found, indicating a statistically significant positive correlation between CBCA concentration and treatment efficacy of spinal disk herniation pain, as can be seen in **FIG. 3C** and summarized in herein above **Table 1.**

No correlation with efficacy was found for THCA (r=0.168, FDR corrected *p-*value=0.993, original *p*-value=0.549), THC (r=0.315, FDR corrected *p*-value= 0.942, original *p*-value=0.25) or Total THC (r=0.264, FDR corrected *p*-value =0.942, original*p*-value=0.343). Similarly, no correlation with efficacy was found for CBDA (r=0.09, FDR corrected *p-*value=0.993, original *p*-value=0.749), CBD (r=-0.356, FDR corrected *p*-value=0.942, original p-value=0.347) or Total CBD (r=0.374, FDR corrected *p*-value=0.942, original*p*-value=0.17).

Interestingly, and counterposed with the varying literature highlighting dose dependent effects of THC and CBD, neither of their acid forms (THCA or CBDA), neutral forms (THC or CBD) and total values taken together (total THC or total CBD) were found to be significantly correlated with the effective treatment of spinal disk herniation.

As each strain was assigned with its median CBCA amount (% concentration (w/w) of a molecule in the dry cannabis plant sample) from all the strain's batches, the 50% (median) and 75% percentiles were calculated based on all the strains medians.

In accordance, a minimal daily dose of 575 ug/day was determined based on the strains concentrations 50% percentile, which was 0.17%, and considering minimal daily consumption of at least about 0.333 grams of cannabis inflorescence by a subject (i.e., 10g per month); and a minimal daily dose of 800ug/day was determined based on the 75% percentile which was 0.24% and considering minimal daily consumption of at least about 0.333 grams by a subject (i.e., 10g per month).

In addition, CBCA to Total THC ratio (calculated as (% CBCA / % Total THC) X 100%) and CBCA to Total CBD ratio (calculated as (% CBCA / % Total CBD) X 100%) was calculated based on their concentrations for each of the 9 strains. The median of this ratio (1.05% rounded to 1.0%) for Total THC and (475%) for Total CBD can be considered a minimum ratio of CBCA to Total THC needed for the efficacious treatment of spinal disk herniation pain.

In conclusion, it was advantageously found that the concentrations of the cluster representing CBCA across the strains were positively correlated, in a statistically significant manner, with the efficacies of these strains in treating/attenuating/reducing/preventing severe spinal disk herniation pain, self-reported by the subjects as level 5 out of 5 i.e., severe pain.

### Example 4 - Efficacy evaluation of potential cannabis compositions in the treatment of fibromyalgia (FM) pain, and identification of CBCA and CBC (total CBC) as cannabis molecules whose combined concentrations correlate with a positive outcome

To evaluate the potential efficacy of the different cannabis molecules in the treatment of additional pain specific conditions and/or symptoms, the clinical and chemical data sets were further analyzed, following the same general working frame, workflow and methods described in the **"Overview of the study presented in Examples 1-6"** and in **Example 1.**

The analysis exemplified herein refers to 49 subjects with fibromyalgia (FM) who self-evaluate their pain specific condition and/or symptom on a scale of 1-5 and ranked it as 5 out of 5 - i.e., severe pain, and were asked to self-evaluate the level of perceived efficacy of their cannabis treatment in reducing the level of their pain on a scale of 1-7, where 1 represents lack of any cannabis effect (i.e., inefficient/ineffective treatment) and 7 represents high efficacy of treatment (i.e., extremely effective).

The patients reported they administer/consume the cannabis by vaping or smoking any of 8 different strains of cannabis, namely Persian Kush, Tel Aviv, Rome, Tchelet Till, Black, Kush Dan, Marmalade, and Flame.

A potential efficacious cannabis molecule's concentration (as measured chemically) should be positively correlated with treatment efficacy (as reported by the patients), across all strains, in a statistically significant manner. Advantageously, using this approach a molecule showing a statistically significant high correlation indicates that as its dosage increases, the treatment of fibromyalgia pain in a population of fibromyalgia patients is more effective.

A Spearman correlation was then used to quantify the correlation between the questionnaire's efficacy of each strain (the label) and the concentration of a specific cannabis molecule in each strain (i.e., the clinical data vs. the chemical data of each of the 19 clusters representing known cannabinoids, including CBC and CBCA). Since 19 clusters were tested, the *p*-values were adjusted using the False Discovery Rate (FDR) method, taking into account multiple tests.

Advantageously and surprisingly, it was found that the concentrations of the clusters representing Total CBC (reference is made to **FIG. 1** and **FIG. 2A****-2B** for the method of identification) across 8 strains were positively correlated with the perceived efficacies of these strains in treating/attenuating/preventing fibromyalgia pain as was self-reported by the subjects.

A Spearman correlation of r=0.89 (p-value=0.025, after FDR correction) was found, indicating a statistically significant positive correlation between Total CBC concentration (divided by the total THC level) and treatment efficacy of fibromyalgia pain, as can be seen in **FIG. 3D** and summarized in herein above **Table 1.**

No correlation with efficacy was found for THCA (r=-0.506, FDR corrected *p-*value=0.502, original *p*-value=0.201), THC (r=0.157, FDR corrected *p*-value=0.732, original *p*-value=0.711) or total THC (r=-0.192, FDR corrected *p*-value=0.733, original *p-*value=0.647). Similarly, no correlation with efficacy was found for CBDA (r=-0.217, FDR corrected *p*-value=0.733, original *p*-value=0.606), CBD (r=-0.41, FDR corrected *p-*value=0.57, original *p*-value=0.313) or total CBD (r=-0.289, FDR corrected *p*-value=0.733, original *p*-value=0.487).

Interestingly, and counterposed with the varying literature highlighting dose dependent effects of THC and CBD, neither of their acid forms (THCA or CBDA), neutral forms (THC or CBD) and total values taken together (total THC or total CBD) were found to be significantly correlated with the effective treatment of fibromyalgia pain.

The complexity of researching cannabis treatment in Israel is due in part to the variety of available strains, THC and CBD levels for each strain, and a variety of consumption methods including smoking, vaping, and orally-used oil. Moreover, patients can consume more than one strain in the same month, with varying doses of each strain. In the current study, patients reported using 12 unique cannabis strains.

In addition, as patients self-titrate cannabis consumption to standardize THC intake (the more THC in the strain, the less patients consume in individual sessions), the data was aimed to decrease this effect by normalizing the concentrations of the studied cannabis molecules in each batch/strain to the total THC amounts of the batch/strain. This was done to achieve a more accurate representation of the amount of each molecule consumed by patients. The concentration of each molecule of each batch was divided by its total THC amount. Total THC amount was calculated, per batch, as THC amount + 0.877 × THCA amount (accounting to full decarboxylation of THCA to THC, as expected in consumption via smoking). The total THC value of each cluster (representing a cannabis molecule) for each of the strains was calculated as the median of the strain's batches.

As each strain was assigned with its median total CBC amount (% concentration (w/w) of a molecule in the dry cannabis plant sample) from all the strain's batches, the 50% (median) and 75% percentiles were calculated based on all the strains medians.

In accordance, a minimal daily dose of 575 ug/day was determined based on the strains concentrations 50% percentile, which was 0.17%, and considering minimal daily consumption of at least about 0.333 grams of cannabis inflorescence by a subject (i.e., 10g per month); and a minimal daily dose of 800ug/day was determined based on the 75% percentile which was 0.24% and considering minimal daily consumption of at least about 0.333 grams by a subject (i.e., 10g per month).

In addition, total CBC to total THC ratio (calculated as (% total CBC / % total THC) X 100%) and total CBC to total CBD ratio (calculated as (% total CBC / % total CBD) X 100%) was calculated based on their concentrations for each of the 8 strains. The median of this ratio (0.8, rounded to 1%) for total THC and (330%) for Total CBD can be considered a minimum ratio of Total CBC to total THC and Total CBD, respectively, needed for the efficacious treatment of fibromyalgia pain.

In conclusion, it was found that the concentrations of the cluster representing total CBC across the strains were positively correlated, in a statistically significant manner, with the efficacies of these strains in treating/attenuating/reducing/preventing severe fibromyalgia pain, self-reported by the fibromyalgia patients as level 5 out of 5 i.e., severe pain).

### Example 5 - Efficacy evaluation of potential cannabis compositions in the treatment of headaches, and identification of CBCA and CBC (total CBC) as cannabis molecule whose combined concentration correlates with a positive outcome

To evaluate the potential efficacy of the different cannabis molecules in the treatment of additional pain specific conditions and/or symptoms, the clinical and chemical data sets were further analyzed, following the same general working frame, workflow and methods described in the **"Overview of the study presented in Examples 1-6"** and in **Example 1.**

The analysis exemplified herein refers to 22 subjects with headaches who self-evaluate their pain specific condition and/or symptom on a scale of 1-5 and ranked it as 5 out of 5 - i.e., severe headaches, and were asked to self-evaluate the level of perceived efficacy of their cannabis treatment in reducing the level of their headaches pain on a scale of 1-7, where 1 represents lack of any cannabis effect (i.e., inefficient/ineffective treatment) and 7 represents high efficacy of treatment (i.e., extremely effective).

The patients reported they administer/consume the cannabis by vaping or smoking any of 10 batches of various strains of cannabis, namely Tel Aviv (batch number 25721FL02), Rome (02721FL01, 07421FL04, 14ROMI2022001, 18921FL01, 22321FL01), Tchelet Till (17121FL01, 20921FL04, 33221FL03), Kush Dan (06721FL02).

A potential efficacious cannabis molecule's concentration (as measured chemically) should be positively correlated with treatment efficacy (as reported by the patients), across all batches/strains, in a statistically significant manner. Advantageously, using this approach a molecule showing a statistically significant high correlation indicates that as its dosage increases, the treatment of headaches is more effective.

A Spearman correlation was then used to quantify the correlation between the questionnaire's efficacy of each batch (the label) and the concentration (divided by the total THC level) of a specific cannabis molecule in each strain (i.e., the clinical data vs. the chemical data of each of the 19 clusters representing known cannabinoids, including CBCA and CBC, together total CBC). Since 19 clusters were tested, the *p*-values were adjusted using the False Discovery Rate (FDR) method, taking into account multiple tests.

Advantageously and surprisingly, it was found that the concentrations of the clusters representing total CBC (reference is made to **FIG. 1** and **FIG. 2A****-2B** for the method of identification) across 10 batches were positively correlated with the perceived efficacies of these batches in treating/attenuating/preventing headaches as was self-reported by the subjects.

A Spearman correlation of r=0.81 (*p*-value=2.9E-02, after FDR correction) was found, indicating a statistically significant positive correlation between total CBC concentration and treatment efficacy of headaches, as can be seen in **FIG. 3E** and summarized in herein above **Table 1.**

No correlation with efficacy was found for THCA (r=-0.178, FDR corrected *p-*value=0.928, original *p*-value=0.622), THC (r=0.702, FDR corrected *p*-value=0.121, original *p*-value=0.024) or Total THC (r=-0.178, FDR corrected *p*-value=0.928, original *p-*value=0.622). Similarly, no correlation with efficacy was found for CBDA (r=0.148, FDR corrected *p*-value=0.928, original *p*-value=0.684), CBD (r=0.204, FDR corrected *p-*value=0.928, original *p*-value=0.571) or Total CBD (r=0.314, FDR corrected *p*-value=0.796, original *p*-value=0.377).

Interestingly, and counterposed with the varying literature highlighting dose dependent effects of THC and CBD, neither of their acid forms (THCA or CBDA), neutral forms (THC or CBD) and total values taken together (total THC or total CBD) were found to be significantly correlated with the effective treatment of headaches.

The complexity of researching cannabis treatment in Israel is due in part to the variety of available strains, THC and CBD levels for each strain, and a variety of consumption methods including smoking, vaping, and orally-used oil. Moreover, patients can consume more than one strain in the same month, with varying doses of each strain. In the current study, patients reported using 12 unique cannabis strains.

In addition, as patients self-titrate cannabis consumption to standardize THC intake (the more THC in the strain, the less patients consume in individual sessions), this study aimed to decrease this effect by normalizing the concentrations of the studied cannabis molecules in each batch/strain to the total THC amounts of the batch/strain. This was done to achieve a more accurate representation of the amount of each molecule consumed by patients. The concentration of each molecule of each batch was divided by its total THC amount. Total THC amount was calculated, per batch, as THC amount + 0.877 × THCA amount (accounting to full decarboxylation of THCA to THC, as expected in consumption via smoking). The total THC value of each cluster (representing a cannabis molecule) for each of the 10 batches was calculated as the median of the strain's batches.

The median (50%) and 75% percentiles total CBC amount (% concentration (w/w) of a molecule in the dry cannabis plant sample) of all the batches was calculated.

In accordance, a minimal daily dose of 1000 ug/day was determined based on the batches concentrations 50% percentile, which was 0.3%, and considering minimal daily consumption of at least about 0.333 grams of cannabis inflorescence by a subject (i.e., 10g per month); and a minimal daily dose of 1300ug/day was determined based on the 75% percentile which was 0.4% and considering minimal daily consumption of at least about 0.333 grams by a subject (i.e., 10g per month).

In addition, total CBC to total THC ratio (calculated as (% total CBC / % total THC) X 100%) and total CBC to total CBD ratio (calculated as (% Total CBC / % total CBD) X 100%) was calculated based on their concentrations for each of the 10 batches. The median of this ratio (1.4% rounded to 1.5%) for THC and 584% (rounded to 550%) for total CBD (calculated in Example 6) can be considered a minimum ratio of Total CBC to total THC and total CBD, respectively, needed for the efficacious treatment of headaches.

In conclusion, it was found that the concentrations of the cluster representing Total CBC across the batches were positively correlated, in a statistically significant manner, with the efficacies of these strains in treating/attenuating/reducing/preventing severe headaches pain, self-reported by the subjects as level 5 out of 5 i.e., severe pain).

### Example 6 - Efficacy evaluation of potential cannabis compositions in the treatment of headaches, and identification of both CBCA and cannflavin A as cannabis molecules whose combined concentrations correlate with a positive outcome

To further evaluate a potential efficacy of additional cannabis molecules, other than CBCA and CBC, in the treatment of headaches, the sets of clinical and chemical data utilized in Example 5 were further analyzed.

This analysis included determining a multiple linear regression model, where the ranked concentrations of two molecules, one of them being CBCA, were the independent variables and the ranked clinical efficacy was the dependent variable. All pairs of the relevant cannabis molecules were scanned that way for highly correlated and statistically significant multiple linear regression models.

The analysis exemplified herein refers to the same 22 subjects described in Example 5, who self-evaluated their pain of headaches as severe (5 out of 5), and self-evaluate the level of perceived efficacy of their cannabis treatment in reducing the level of their pain headaches on a scale of 1-7.

A potential efficacious combination of cannabis molecules concentrations (as measured chemically) should be positively correlated with treatment efficacy (as reported by the patients), across all batches, in a statistically significant manner. Advantageously, using this approach a combination of molecules showing a statistically significant high correlation indicates that as their dosages increase the treatment of headaches is more effective.

Advantageously and surprisingly, it was found that a multiple linear regression model where the concentrations of the clusters representing cannflavin A and CBCA are the independent variables was positively correlated with the perceived efficacies of the 10 batches detailed in Example 5 in treating/attenuating/preventing severe headaches.

The flavonoid Cannflavin A was identified as Cannflavin A based on the following:
The peaks comprising this cluster have a RRT range of 0.57-0.60 whereas the RRT of the cannflavin A standard (provided by Cayman Chemical, item no. 21970, batch no. 0638046) is 0.60 (cannflavin A RT is 8.227, THC RT is 13.610) in the specific HPLC method applied in this study and is shown in **FIG. 4B** demonstrating a retention time identity and growth in Cannflavin A area in the spiked sample in direct proportion to the amount of standard added.

The regression model had an adjusted r² of 0.85 (*p*-value=6e-4, compared with a reduced significance level of 1e-3 due to multiple testing), co-efficient of +0.603 for Cannflavin A rank (p-value=3e-3) and +0.524 for CBCA (*p*-value=7e-3) and a constant of - 0.698 (i.e. the regression model is: efficacy = 0.603×Cannflavin A + 0.524×CBCA - 0.698), as can be seen in **FIG. 4A** and summarized in herein above **Table 1**.

As each strain was assigned with its median cannflavin A and CBCA amounts (% concentration (w/w) of a molecule in the dry cannabis plant sample) from all the strain's batches, the 50% (median) and 75% percentiles were calculated based on all the batches medians.

In accordance, for CBCA a minimal daily dose of 1000 ug/day was determined based on the strains concentrations 50% percentile, which was 0.3%, and considering minimal daily consumption of at least about 0.333 grams of cannabis inflorescence by a subject (i.e., 10g per month); and a minimal daily dose of 1300/day was determined based on the 75% percentile which was 0.4% and considering minimal daily consumption of at least about 0.333 grams by a subject (i.e., 10g per month).

In accordance, for cannflavin A a minimal daily dose of 50 ug/day was determined based on the strains concentrations 50% percentile, which was 0.015%, and considering minimal daily consumption of at least about 0.333 grams of cannabis inflorescence by a subject (i.e., 10g per month); and a minimal daily dose of 60ug/day was determined based on the 75% percentile which was 0.018% and considering minimal daily consumption of at least about 0.333 grams by a subject (i.e., 10g per month).

In addition, Cannflavin A to total THC ratio and CBCA to total THC ratio were calculated for each of the 10 batches. In some embodiments, the medians of these ratios (0.07% and 1.4%, respectively) or the 75% of these ratios (0.09% and 2,3%, respectively) can be considered as minimum ratios of cannflavin A and CBCA, respectively, to total THC needed for the efficacious treatment of severe headaches. In addition, Cannflavin A to total CBD ratio and CBCA to total CBD ratio were calculated for each of the 10 batches. In some embodiments, the medians of these ratios (25% and 550%, respectively) or the 75% of these ratios (30% and 700%, respectively) can be considered as minimum ratios of cannflavin A and CBCA, respectively, to total CBD needed for the efficacious treatment of severe headaches.

Interestingly, and counterposed with the varying literature highlighting dose dependent effects of THC and CBD, they (total THC or total CBD) were not found to be significantly linked with the effective treatment of severe headaches.

In conclusion, it was found that the concentrations of the clusters representing CBCA and cannflavin A across the batches were positively correlated together, in a statistically significant manner, with the efficacies of these strains in treating/attenuating/reducing/preventing headaches self-reported by the subjects as level 5 out of 5 i.e., severe pain).

### Example 7 - Clinical trial for evaluation of novel standardized cannabis extract formulations including CBC and cannflavin A for the treatment of pain in fibromyalgia (FM) patients.

The following study is conducted according to the laws and regulations governing medical product clinical trials in Israel (according to the Israeli Ministry of Health regulations) and GCP standards.

### Protocol synopsis:

| | |
|---|---|
| **Study Title** | Response to treatment with cannabis oil in individuals with fibromyalgia |
| **Type of study** | A prospective randomized, controlled double-blind study |
| **Study Intervention** | **Novel formulation/investigational product:** Oral liquid of standardized cannabis extract diluted in MCT oil at a concentration of 50mg/ml THC, 50mg/ml CBD, 0.24mg/ml cannflavin A and 2.0mg/ml CBC. |
| | The maximal daily dose of THC is set to 40mg, corresponding to 192ug of cannflavin A per daily dose, in line with the predetermined minimal amount/concentration of at least 50ug cannflavin A per daily dose. To reach this minimal daily amount of cannflavin A 0.21ml of oil, in which there are 10.4mg of THC, is enough. This minimal amount is expected to be reached by most, if not all, of the subjects during titration (see below). |
| | The maximal daily dose of THC is set to 40mg, corresponding to 1600ug of CBC per daily dose, in line with the predetermined minimal amount/concentration of at least 575ug CBC per daily dose. To reach this minimal daily amount of CBC 0.29ml of oil, in which there are 14.4mg of THC, is enough. This minimal amount is expected to be reached by most, if not all, of the subjects during titration (see below). |
| | **Control:** placebo oil (oral) including cannflavin A and CBC at a concentration below their minimally predetermined amount, or preferably without any cannflavin A or CBC. |
| **Study Objectives** | **Primary Objective** |
| | To evaluate the efficacy of the herein disclosed investigational product including at least predetermined minimal amount/concentration of cannflavin A and CBC, compared to control, for treatment of pain in a population of fibromyalgia (FM) patients. |
| **Inclusion Criteria** | 1. Adult women aged 30 years and over. |
| | 2. Diagnosis of FM confirmed according to the 2016 diagnostic criteria by a pain specialist, with relevant symptoms lasting 12 months or more. |
| | 3. An average reported pain ≥ 6 during the preceding week. |
| | 4. Eligible for cannabis at the discretion of the treating physician. |
| | 5. Has remained symptomatic despite receiving standard care for fibromyalgia. |
| | 1. Patients who have used cannabis during the preceding month. |
| | 2. Any significant comorbid condition at the discretion of the PI (e.g., inflammatory arthritis, inflammatory bowel disease, cancer). |
| | 3. Personal or family history of psychotic disorders. |
| | 4. Current or past anxiety disorder (Hospital anxiety and depression scale) HADS (≥7). |
| **Exclusion Criteria** | 5. Any uncontrolled psychiatric pathology. |
| | 6. Current or history of substance addiction or abuse. |
| | 7. Diagnosed dementia or cognitive impairment. |
| | 8. Personal history of cardiovascular disease. |
| | 9. Pregnancy, lactation or intention to conceive. |
| | 10. Known allergy to any of the cannabis oil ingredients. |
| | 11. Patients with a history of seizure disorder (excluding childhood febrile convulsions) or epilepsy. |
| | 12. Active liver disease. |
| | 13. Any contraindication to the use of MC. |
| | The study is designed to have prospective randomized, controlled double blind design. |
| | **Clinic visit 1: patient recruitment - screening visit** |
| | Candidate FM patients are offered to participate in the study in one of the following ways: |
| | 1) by their treating physician (at Rambam Institute for Pain Medicine). |
| | 2) by advertisement in the local media, dedicated website and social media. |
| | Candidate FM patients are given detailed explanation of the study aims, procedures, potential benefits and risks. After signing an informed consent form, participants undergo a medical evaluation by a certified pain physician to validate the diagnosis of fibromyalgia based on the 2016 diagnostic criteria and to determine eligibility for treatment with MC. |
| **Study Design** | participants: |
| | 1. Fill in the study questionnaires: |
| | • Short-form health-questionnaire (SF12) - a 12-item measure of health-related quality of life, validated in a variety of population groups (24). |
| | • Pittsburgh Sleep Quality Index (PSQI) - a standardized self-administered questionnaire for the assessment of subjective sleep quality (25). |
| | • Hospital anxiety and depression scale (HADS) - questionnaire which is commonly used to determine the levels of anxiety and depression that a person is experiencing (26). |
| | • Fibromyalgia Impact Questionnaire (FIQ) - for the assessment of the impact of fibromyalgia on quality of life, the score ranges from 0 to 100. The higher the score, the greater the impact of FM on individuals' quality of life (27). |
| | • Pain intensity on a numeric rating scale (0-10) describing the maximal, minimal and average pain over the previous 24 hours and the average pain over the previous week. |
| | 2. Give a blood sample for metabolomics; cannabinoid receptor expression (2 tubes, 10 mL total). |
| | 3. Asked about their previous use of cannabis and instructed not use any other type of cannabis during the study. |
| | 4. Instructed not to make any changes in ongoing pain treatment (pharmaceutical or other) during the study. |
| | **Eligible participants:** |
| | Eligible subjects complying with all inclusion criteria (including an average reported pain ≥ 6 during the preceding week) and none of the exclusion criteria are enrolled in the study after signing an informed consent form. |
| | **Clinic visit 2** - **randomization and treatment assignment** |
| | 1. Participants deliver genomic and metabolomics stool samples. |
| | 2. Participants subsequently be randomized to either the active treatment or placebo arm in a ratio of 4:1. They are instructed as for the titration of the cannabis oil. |
| | 3. The investigational product (or the control placebo) is dispensed for the entire duration of the study (3 months). Bottles are labelled such that the patients, physicians and researchers are blinded to their contents (investigational productor placebo). |
| | **Titration** |
| | Participants receive an initial daily dose of 4 mg THC/CBD (equivalent of 1 drop). Participants are instructed to increase the daily dosage every day with an additional 4 mg THC/CBD divided to three daily doses, until reaching an adequate analgesic effect with tolerable side effects or until reaching the maximal daily dose of 40 mg THC/CBD. Titration does not exceed 4-6 weeks until reaching a stable dose. |
| | **Follow up** |
| | **Phone interview** |
| | Participants are contacted by phone every 7 (±3) days during the titration period and every 2 weeks (±3) days after reaching a stable dose and are: |
| | 1. Asked about their pain intensity (maximal, minimal and average) during the preceding 24 hours and about their average pain over the previous week. |
| | 2. Asked about potential side effects following the treatment. |
| | 3. Asked of changes in ongoing analgesic treatments, analgesic consumption and antibiotic consumption. |
| | 4. Fill in the Patient Global Impression of Change (PGIC) - a 7-point scale depicting the patient's belief about the efficacy of treatment (28). |
| | **Clinic visit 3 - after a 3-month treatment period** |
| | Participants: |
| | 1. Fill in the study questionnaires (pain, anxiety, depression, sleep difficulties, and quality of life scales). |
| | 2. Asked if they started receiving antibiotics since the last phone call. |
| | 3. Give a blood sample for: metabolomics; cannabinoid receptor expression; serum levels of cannabinoid metabolites (3 tubes, 15 mL total). |
| | 4. Study code is unblinded. Participants in the placebo arm are given the opportunity to receive the investigational product (i.e., cannabis oil) for three months. Participants who choose to receive the treatment undergo the same procedures and follow up as specified above. |
| **Outcome Measures** | **Efficacy assessment:** |
| | Efficacy assessment is based on evaluation of the change in pain intensity and in other clinical measures (i.e., sleep difficulties, quality of life, anxiety, and depression) based on comparison between the study questionnaires, including Short-form health-questionnaire (SF12), Pittsburgh Sleep Quality Index (PSQI), Hospital anxiety and depression scale (HADS), Fibromyalgia Impact Questionnaire (FIQ), and Pain intensity on a numeric rating scale (0-10), filled at the screening visit and following 3 months of treatment with the investigational product or the control.. |
| **Study Population** | 150 women aged >30 years old, diagnosed with fibromyalgia (confirmed by the modified 2016 ACR Diagnostic Criteria for Fibromyalgia) |
| **Regulatory and Ethical Aspects** | The study is conducted according to the laws and regulations governing medical product clinical trials in Israel (according to the Israeli Ministry of Health regulations) and GCP standards. |

### Example 8 - Clinical trial for evaluation of novel standardized cannabis extract formulations for the treatment of pain in oncology patients

The following study is conducted in accordance with the Faculty Research Ethics Committee and the University of Malta Research Ethics Committee, at the Sir Anthony Mamo Oncology Centre, Msida, Malta.

### protocol synopsis:

| | | | |
|---|---|---|---|
| **Title** | Use of Cannflavin A- and CBC-Rich Cannabis Oil in Improving Quality of Life in Oncology Patients | | |
| **Study description** | Oncology patients suffering from pain are administered with the herein disclosed novel formulation of investigational product. Improvement in symptoms related to anxiety, pain, sleep difficulties and improvement in quality of life of patients is assessed. | | |
| **Study Intervention** | **Novel formulation / investigational product:** | | |
| | Oral liquid of standardized cannabis extract diluted in oil at a concentration of 25mg/ml Δ-9-tetrahydrocannabinol (THC), 25mg/ml cannabidiol (CBD), and at least 0.05 mg/ml cannflavin A and 1.0 mg/ml CBC | | |
| | The maximal daily dose of THC is set to 40mg, corresponding to 80ug of cannflavin A per daily dose, and 1600 ug of CBC per daily dose which corresponds to the predetermined minimal amount/concentration of at least 40ug cannflavin per daily dose and at least 575ug CBC per daily dose. | | |
| | **Control:** placebo oil (oral) including cannflavin A and CBC at concentration below its minimally predetermined amount, or without any cannflavin A or CBC. | | |
| **Study population** | Oncology patients suffering from pain | | |
| **Primary objective** | To evaluate the efficiency of the herein disclosed investigational product including at least predetermined minimal amount/concentration of cannflavin A and CBC compared to control, for the treatment of pain in oncology patients. | | |
| | Endpoint: Improvement (i.e. less pain) in the Pain intensity on a numeric rating scale (0-10). | | |
| | Endpoint: Decreases in dosages of conventional analgesic medication. | | |
| **Secondary objective** | | 1. Evaluating the efficacy of the herein disclosed standardized cannabis formulation for the improvement in the quality of life of oncology patients. | |
| | | | - Endpoint: Improvement in the Eastern Cooperative Oncology Group (ECOG) Performance Status Scale and QOL10 (Ventegodt et al.) |
| | | 2. Evaluating the efficacy of the herein disclosed standardized cannabis formulation in the treatment of sleep difficulties. | |
| | | | - Endpoint: Improvement in Insomnia Severity Index (ISI) |
| | | 3. Evaluating the efficacy of the herein disclosed standardized cannabis formulation in the treatment of anxiety and depression | |
| | | | - Endpoint: Improvement in Patient Health Questionnaire-4 (PHQ-4) |
| | | | - Endpoint: Improvement in Generalized Anxiety Disorder 7-item Scale (GAD-7) |
| | | 4. Evaluating the overall perception of improvement and treatment satisfaction of the herein disclosed standardized cannabis formulation | |
| | | | - Endpoint: Improvement in Patient Global Impression of Change (PGIC) |
| | | 5. Evaluating the safety of the herein disclosed standardized cannabis formulation in the treatment of pain in oncology patients | |
| | | | - Endpoint: Rates of Adverse Events (AEs) based on weekly telephone calls made by physician. |
| | | 6. Establishing recommendation for dosage regimen in the treatment of pain. | |
| | Endpoint: Finding the optimal dose for treatment. Based on data collected regarding dosage, efficacy, and AE, by physician observations during visits/calls, dosage recommendations will be developed. | | |
| **Sample size** | 60 patients | | |
| **Duration of study treatment** | 10 weeks | | |
| **Unique aspects of the study** | Study of the effectiveness of cannflavin A and CBC in standardized cannabis preparations, optimal dosing regimens and routes of administration in various patient groups. | | |
| **Study design** | The study is a randomized, prospective, add-on, controlled, double-blind design and will include at least 60 oncology patients naive to medical cannabis (i.e., not having used cannabis at all or at least not within the last month) with previously diagnosed pain. | | |
| | The study is carried out in two (2) periods (as illustrated in **FIG. 5****)** according to the hereinbelow described time schedule. | | |
| | **Screening and enrollment visit** - 1 day | | |
| | Explanation and description of the study. | | |
| | Eligible subjects complying with all inclusion criteria and none of the exclusion criteria are enrolled in the study after signing an informed consent form including waiver in case of patient experiencing any product adverse effects | | |
| **Study design** | • Physical examination, blood count, blood pressure, heart rate, vital signs, and medical history. | | |
| | • Participants are asked to provide demographic information. | | |
| | • At the screening visit and the end of each study period, participants are required to complete a questionnaire assessing measures of pain and other indications (i.e., anxiety, sleep difficulties) related to the effectiveness of the current treatment. Participants are assigned to fill out the questionaries including: | | |
| | | | • Numeric Pain Rating Scale (NPRS) |
| | | | • Pain Self-Efficacy Questionnaire (PSEQ) |
| | | | • Patient Health Questionnaire-4 (PHQ-4) |
| | | | • Generalized Anxiety Disorder 7-item Scale (GAD-7) |
| | | | • Insomnia Severity Index (ISI) |
| | | | • QOL 10 (Ventegodt et al.) |
| | | | • Patient Global Impression of Change (PGIC) |
| | Physician will assess patients using Eastern Cooperative Oncology Group (ECOG) Performance Status Scale | | |
| | Women will be required to have a negative urine pregnancy test result. | | |
| | Enrolled participants will continue treatment with previously prescribed products for treatment of pain. | | |
| | Participants are randomized in a double-blind manner to attend one of two study arms of treatment: participants in one arm receives treatment with the herein disclosed novel standardized cannabis extract (investigational product), and participants in the other arm are treated with a control product / the placebo, as described above). Subjects are asked to avoid the use of any other type of cannabis products during the study. | | |
| | **Period one** - 6 weeks- Dose titration of assigned product. | | |
| | **Period two** - 4 weeks- Participants remain on a stable dose. | | |
| | ***Follow-up calls*** - during period one and period two. Weekly phone calls by PI that include reporting the current dose of the investigational product, the occurrence of adverse events, and changes in concomitant medications. Patients report adverse effects, dosage, concomitant medications to physician | | |
| | Physician will assess patients using Eastern Cooperative Oncology Group (ECOG) Performance Status Scale Following tools will be filled in | | |
| | | | - Numeric Pain Rating Scale (NPRS) |
| | | | - Patient Global Impression of Change (PGIC) |
| | **Termination visit (Visit 2)-** 1 day | | |
| | Physical examination, blood count, blood pressure, heart rate, vital signs, and medical history | | |
| | Enrolled subjects will fill-in the study questionnaires: | | |
| | • Numeric Pain Rating Scale (NPRS) | | |
| | • Pain Self-Efficacy Questionnaire (PSEQ) | | |
| | • Patient Health Questionnaire-4 (PHQ-4) | | |
| | • Generalized Anxiety Disorder 7-item Scale (GAD-7) | | |
| | • Insomnia Severity Index (ISI) | | |
| | • QOL10 (Ventegodt et al.) | | |
| | • Patient Global Impression of Change (PGIC) | | |
| | Physician will assess patients using Eastern Cooperative Oncology Group (ECOG) Performance Status Scale | | |
| | Study is a single-site study- Sir Anthony Mamo Oncology Centre, Msida, Malta | | |
| **Outcome Measures** | **Efficacy Assessment:** | | |
| | Efficacy assessment is carried out by comparing scores measured with the questionnaire at the screening visit and at the end of each study period. | | |
| **Safety assessment** | Throughout the study period, patients are asked to complete a short daily online form that includes reporting the current dose of the investigational products, occurrence in adverse events and changes in concomitant medications. | | |
| | Adverse events are graded according to the National Cancer Institute Common Terminology Criteria for Adverse Events, version 5.0 (US Department of Health and Human Services, 2017). | | |
| **Withdrawals/ Discontinuation of the study** | Subjects prematurely discontinued from the investigation after the beginning of *treatment period* will perform an 'Early termination visit', in which procedures scheduled for *'Termination visit'* will be performed (whenever feasible). In case of early termination, the Investigator will duly record the reason for premature withdrawal in the dedicated section of the CRF. | | |
| **Inclusion criteria** | | | • Adult males or females aged 25 years or older having cancer, suffering from pain |
| | | | • Patients having at least another 6 months to live, as assessed by physician |
| | | | • Participants should confirm abstention from the recreational or medical use of any cannabis products for at least one year. Participants must agree to abstain from recreational cannabis use for the duration of the study |
| | | | • Concomitant analgesic medication use will be allowed, provided that the dose has been stable for at least4 weeks prior to randomization (including opioids, NSAIDs) |
| | | | • The ability to comprehend and satisfactorily comply with protocol requirements |
| | | | • Women of childbearing potential will be required to have a negative urine pregnancy test result. |
| | | | • Written informed consent given prior to entering the baseline period of the study |
| | | | • Speaking Maltese or English |
| **Exclusion criteria** | | | • Serious, unstable medical condition at the discretion of the PI. Including but not limited to cerebrovascular, renal, hepatic, coronary heart disease, coagulation or blood disorders, use of anticoagulant medication, pre-existing neurological illness, pre-existing cardiovascular disease including poorly controlled hypertension, ischemic heart disease, arrhythmia or heart failure. |
| | | | • History of bipolar disorder, psychotic disorder or schizophrenia, schizoaffective disorder, obsessive-compulsive disorder, or personality disorder (Cluster A or B) |
| | | | • Cannabis use or use of medications or drugs targeting the endocannabinoid system including but not limited to nabiximols, nabilone, or synthetic cannabinoids over the past year |
| | | | • Pregnancy or lactation. Males and females of child-bearing potential must be using and willing to continue using medically acceptable contraception throughout the study to avoid pregnancy during the study and for up to 4 weeks after study completion. Study acceptable methods of birth control are double-barrier methods, which include a combination of any 2 of the following: oral contraceptives, diaphragm, condom, copper intrauterine device, sponge, spermicide, or (partner's) vasectomy |
| | | | • Reported history of difficulty with intravenous blood draws |
| | | | • Allergy to or intolerability of cannabinoids, or other ingredients of the product |
| | | | • Currently undergoing active treatment e.g. chemotherapy, radiotherapy and immunotherapy treatment |
| | | | • Patients having less than 6 months to live |
| **Reporting and documentation of results** | Main researcher collects pseudo-anonymized data and study statistician reviews and analyzes anonymized data. Data is encrypted and password protected. Data is destroyed at the end of the study period. | | |
| **Ethical Aspects** | The study is conducted in accordance with the Faculty Research Ethics Committee and the University of Malta Research Ethics Committee. | | |

| **Period** | **Duration** | **Description** |
|---|---|---|
| Study design - Screening visit and baseline | 1 day | Explanation and description of the study Eligible patients are enrolled in study after filling in informed consent. |
| | | Physical examination, blood count, blood pressure, heart rate, vital signs and medical history |
| | | Participants are asked to provide demographic information. |
| | | Women are required to have a negative urine pregnancy test result. |
| | | Enrolled participants continue treatment with previously prescribed products for treatment of pain throughout the length of the study. |
| | | Participants are randomized in a double-blind manner to attend one of the 2 study arms to receive investigational product or placebo. |
| | | The pain intensity Score (and other questionnaire scores)are collected. |
| | | The ECOG Performance Status Scale is collected. |
| Study design - Period 1 | 6 weeks | Dose titration of assigned product |
| | | The pain intensity Score and other questionnaire scores are collected during the weekly follow-up calls. |
| | | The ECOG Performance Status Scale is collected. |
| Study design - Period 2 | 4 weeks | Dose titration. |
| | | Patients remaining on stable dose of assigned product. |
| | | The pain intensity Score and other questionnaire scores are collected during the weekly follow-up calls. |
| | | The ECOG Performance Status Scale is collected. |
| Study design - Termination visit | | Physical examination, blood count, blood pressure, heart rate, vital signs and medical history |
| | | The pain intensity Score (and other questionnaire scores) are collected. |
| | | The ECOG Performance Status Scale is collected. |

### Example 9 - Medical Cannabis Products

The following are non-limiting examples of formulation and ways of obtaining them, they should in no way be construed as limiting the broad scope of the invention.
**Example of formulation 1:** Standardized extract of 26mg/g (25mg/mL) THC, 26mg/g (25mg/mL) CBD, 3mg/g CBC and 0.15 mg/g Cannflavin A.
   Two extracts are used: a THC rich extract as a source of both THC, CBC and Cannflavin A (extract A: 70%w/w THC, 0.5%w/w CBD, 0.59% CBC and 0.42%w/w Cannflavin A) and a CBD rich extract as a main source of CBD and CBC (extract B: 2%w/w THC, 65%w/w CBD, 7.0%w/w CBC and 0%w/w Cannflavin A).
   To obtain 10,000g of the standardized extract, 360g of extract A is mixed with 398g of extract B, 9192g of MCT oil and 50g of Vitamin E.
   Alternatively, to obtain 33,000g of the standardized extract, 1188g of extract A is mixed with 1313.4g of extract B, 30333.6g of MCT oil and 165g of Vitamin E.
   The resulting 10,000g and 33,000g standardized extracts contain 26mg/g of THC, 26mg/g of CBD, 3mg/g CBC and 0.15 mg/g Cannflavin A.
   Formulation 1 in %(w/w): Standardized extract of 2.6% (w/w) THC, 2.6% (w/w) CBD, 0.3% (w/w) total CBC, 0.015% (w/w) Cannflavin A and optionally 0.5% (w/w) Vitamin E.
**Example of formulation 2:** Standardized extract of 52mg/g (50mg/mL) THC, 52mg/g (50mg/mL) CBD, 6mg/g CBC and 0.3 mg/g Cannflavin A.
   The same two extracts from example formulation 1 are used.
   To obtain 10,000g of the standardized extract, 720g of extract A is mixed with 796g of extract B, 8434g of MCT oil and 50g of Vitamin E.
   Alternatively, to obtain 25,000g of the standardized extract, 1800g of extract A is mixed with 1990g of extract B, 21085g of MCT oil and 125g of Vitamin E.
   The resulting 10,000g and 25,000g standardized extracts contain 52mg/g of THC, 52mg/g of CBD, 6mg/g CBC and 0.3 mg/g Cannflavin A.
   Formulation 2 in %(w/w): Standardized extract of 5.2% (w/w) THC, 5.2% (w/w) CBD, 0.6% (w/w) CBC, 0.03% (w/w) Cannflavin A and optionally 0.5% (w/w) Vitamin E.
**Example of formulation 3:** Standardized extract of 26mg/g (25mg/mL) THC, 26mg/g (25mg/mL) CBD and 1.7mg/g CBC.
   Two extracts are used: a THC rich extract as a source of both THC and CBC (extract A: 75%w/w THC, 0.6%w/w CBD and 0.46% CBC) and a CBD rich extract as a main source of CBD and CBC (extract B: 2.5%w/w THC, 60%w/w CBD, 3.6%w/w CBC).
   To obtain 10,000g of the standardized extract, 332g of extract A is mixed with 430g of extract B, 9188g of MCT oil and 50g of Vitamin E.
   To obtain 31,000g of the standardized extract, 1030g of extract A is mixed with 1333g of extract B, 28482g of MCT oil and 155g of Vitamin E.
   The resulting 10,000g and 31,000g standardized extracts contain 26mg/g of THC, 26mg/g of CBD and 1.7mg/g CBC.
   Formulation 3 in %(w/w): Standardized extract of 2.6% (w/w) THC, 2.6% (w/w) CBD, 0.17% (w/w) CBC and optionally 0.5% (w/w) Vitamin E.
**Example of formulation 4:** Standardized extract of 52mg/g (50mg/mL) THC, 52mg/g (50mg/mL) CBD and 3.4mg/g CBC.
   The same two extracts from example formulation 3 are used.
   To obtain 10,000g of the standardized extract, 665g of extract A is mixed with 860g of extract B, 8425g of MCT oil and 50g of Vitamin E.
   To obtain 22,000g of the standardized extract, 1463g of extract A is mixed with 1892g of extract B, 18535g of MCT oil and 110g of Vitamin E.
   The resulting 10,000g and 22,000g standardized extracts contain 52mg/g of THC, 52mg/g of CBD and 3.4mg/g CBC.
   Formulation 4 in %(w/w): Standardized extract of 5.2% (w/w) THC, 5.2% (w/w) CBD, 0.34% (w/w) CBC and optionally 0.5% (w/w) Vitamin E.
**Example of formulation 5:** Quantified extract as API for a transdermal drug delivery system (TDDS) formulation of 368 mg/g THC, 368 mg/g CBD, 2.7 mg/g Cannflavin A and 24 mg/g CBC.
   To obtain 2,000g of the quantified extract, two extracts are used: a Cannflavin A enriched THC rich extract as a source of both THC and Cannflavin A (extract A: 90%w/w THC, 0.72%w/w CBD, 0.69% w/w Cannflavin A and 0.55%w/w CBC) and a CBD rich extract as a main source of CBD and CBC (extract B: 2.5%w/w THC, 60%w/w CBD, 3.6%w/w CBC). 783.5g of extract A is mixed with 1216.5g of extract B.
   The resulting 2,000g quantified extract contains 368 mg/g of THC, 368 mg/g of CBD, 2.7 mg/g Cannflavin A and 24 mg/g CBC.
   Formulation 5 in %(w/w): Quantified extract of 36.8% (w/w) THC, 36.8% (w/w) CBD, 0.27% (w/w) Cannflavin A and 2.4% (w/w) CBC
**Example of formulation 6:** Quantified extract as API for a transdermal drug delivery system (TDDS) formulation of 378 mg/g THC, 378 mg/g CBD, 15 mg/g Cannflavin A and 30 mg/g CBC.
   To obtain 5,000g of the quantified extract, two extracts are used: a Cannflavin A enriched THC rich extract as a source of both THC and Cannflavin A (extract A: 90%w/w THC, 0.72%w/w CBD, 3.7% w/w Cannflavin A and 0.63%w/w CBC) and a CBD rich extract as a main source of CBD and CBC (extract B: 2.2%w/w THC, 63%w/w CBD, 4.6%w/w CBC).
   2025.5g of extract A is mixed with 2974.5g of extract B.
   The resulting 5,000g quantified extract contains 378 mg/g of THC, 378 mg/g of CBD, 15 mg/g Cannflavin A and 30 mg/g CBC.
   Formulation 6 in %(w/w): Quantified extract of 37.8% (w/w) THC, 37.8% (w/w) CBD, 1.5% (w/w) Cannflavin A and 3.0% (w/w) CBC
**Example of formulation 7:** Standardized extract of 26mg/g (25mg/mL) THC, 26mg/g (25mg/mL) CBD, 1 mg/g CBC.
   To obtain 10,000g of the standardized extract, two extracts are used: a THC rich extract as a source of both THC and CBC (extract A: 70%w/w THC, 0.5%w/w CBD, 0.19%w/w CBC) and a CBD rich extract as a main source of CBD and CBC (extract B: 2%w/w THC, 65%w/w CBD, 2.35%w/w CBC).
   360g of extract A is mixed with 398g of extract B, 9192g of MCT oil and 50g of Vitamin E.
   The resulting 10,000g standardized extract contains 26mg/g of THC, 26mg/g of CBD and 1 mg/g CBC.
   Formulation 7 in %(w/w): Standardized extract of 2.6% (w/w) THC, 2.6% (w/w) CBD, 0.1% (w/w) CBC and optionally 0.5% (w/w) Vitamin E.
**Example of formulation 8:** Standardized extract of 26mg/g (25mg/mL) THC, 26mg/g (25mg/mL) CBD, 1mg/g CBC and 0.087 mg/g Cannflavin A.
   To obtain 10,000g of the standardized extract, two extracts are used: extract A (from example formulation 1) and a CBD rich extract as a main source of CBD, CBC and cannflavin A (extract B2: 2%w/w THC, 65%w/w CBD, 2.35%w/w CBC, 0.22%w/w cannflavin A).
   360g of extract A is mixed with 398g of extract B, 9192g of MCT oil and 50g of Vitamin E.
   The resulting 10,000g standardized extract contains 26mg/g of THC, 26mg/g of CBD, 1 mg/g CBC and 0.087 mg/g Cannflavin A.
   Formulation 8 in %(w/w): Standardized extract of 2.6% (w/w) THC, 2.6% (w/w) CBD, 0.1% (w/w) CBC, 0.0087% Cannflavin A and optionally 0.5% (w/w) Vitamin E.
**Example of formulation 9:** Standardized extract of 52mg/g (50mg/mL) THC, 52mg/g (50mg/mL) CBD, 2mg/g CBC.
   To obtain 10,000g of the standardized extract, the same two extracts from example formulation 1 are used.
   720g of extract A is mixed with 796g of extract B, 8434g of MCT oil and 50g of Vitamin E.
   The resulting 10,000g standardized extract contains 52mg/g of THC, 52mg/g of CBD and 2 mg/g CBC.
   Formulation 9 in %(w/w): Standardized extract of 5.2% (w/w) THC, 5.2% (w/w) CBD, 0.2% (w/w) CBC and optionally 0.5% (w/w) Vitamin E.
**Example of formulation 10:** Standardized extract of 52mg/g (50mg/mL) THC, 52mg/g (50mg/mL) CBD, 2mg/g CBC and 0.174 mg/g Cannflavin A.
   To obtain 10,000g of the standardized extract, the same two extracts from example formulation 2 are used.
   720g of extract A is mixed with 796g of extract B, 8434g of MCT oil and 50g of Vitamin E.
   The resulting 10,000g standardized extract contains 52mg/g of THC, 52mg/g of CBD, 2mg/g CBC and 0.174 mg/g Cannflavin A.
   Formulation 10 in %(w/w): Standardized extract of 5.2% (w/w) THC, 5.2% (w/w) CBD, 0.2% CBC, 0.0174% (w/w) Cannflavin A and optionally 0.5% (w/w) Vitamin E.

Non-limiting formulations 7-10 assumes consumption of at least 575mg product per day (formulation 7-8) or at least 287mg product per day (formulation 9-10), which includes CBCA or total CBC consumption of at least the predetermined minimal amount/concentration of 575ug per daily dose and cannflavin A consumption (for formulations 8 & 10) of at least the predetermined minimal amount/concentration of 50ug per daily dose.

In addition, by assuming consumption of at least twice the amount of product per day (i.e., at least 1.15g (formulation 7-8) or at least 0.575g (formulation 9-10) product per day) non-limiting formulations 7-10 also represent an enriched cannabis extract that includes at least twice the predetermined minimal amounts/concentrations of CBC and cannflavin A per daily dose (i.e., at least 1.15mg and 0.1mg, respectively).

Non-limiting formulations 1-4 and 7-10 include total THC and total CBD concentration of about 2.6%-5.2%w/w each, however, these should not limit the concentration of total THC and total CBD in standardized extract/cannabis composition of the invention which in some embodiments, can include about 10%, about 15%, about 20% 2 about 5%, about 30%, about 35%, about 40%, about 45%, or about 50%. Each possibility is a separate embodiment.

Non-limiting formulations 1-10 also include representation of concentrations in % (w/w), however these are not affected by the amount consumed, do not correlate directly to the predetermined minimal amount, and with respect to CBCA and/or CBC the concentration, in some embodiments may range between 0.00001% CBCA and/or CBC and up to a maximal concentration of about 20.00% or about 10.00% in standardized extract/cannabis composition of the invention.

To conclude, according to some embodiments, non-limiting formulations 1-10 represent compositions of standardized extract fulfilling at least one of any one of the requirements of a cannabis composition comprising (i) the predetermined minimal amount/concentration of at least 575ug CBCA and/or CBC per daily dose; (ii) CBCA and/or CBC to THC concentrations at the minimal characteristic ratio of at least about (0.8%), required for efficacious treatment of any one of the pain-related conditions and/or symptom of the present invention; (iii) enriched amount of CBCA and/or CBC of at least twice the predetermined minimal amount/concentration; and (iv) in some embodiments, it may include cannflavin A at amount of at least 50ug per daily dose.

While certain embodiments of the invention have been illustrated and described, it will be clear that the invention is not limited to the embodiments described herein. Numerous modifications, changes, variations, substitutions and equivalents will be apparent to those skilled in the art without departing from the spirit and scope of the present invention as described by the claims, which follow.

## Claims

1. A cannabis composition comprising a predetermined minimal amount/concentration of at least 575ug CBCA and/or CBC per daily dose, for use in treatment of pain in a subject in need thereof.

2. The cannabis composition for use of claim 1, wherein the predetermined minimal amount of CBCA and/or CBC is at least 800ug, per daily dose, preferably wherein the predetermined minimal amount/concentration of CBCA and/or CBC is at least 1000ug, per daily dose.

3. The cannabis composition for use of claim 1-2, wherein the cannabis composition further comprises cannflavin A at an amount of at least 50ug, per daily dose.

4. The cannabis composition for use of any one of claims 3, wherein the composition comprises cannflavin A at a concentration **characterized by** cannflavin A to total THC ratio of at least about 0.07%, preferably at least about 0.8%.

5. The cannabis composition for use of any one of claims 1-4, wherein CBCA and/or CBC is added to the composition if the CBCA and/or CBC content is below the predetermined minimal amount/concentration.

6. The cannabis composition for use of any one of claims 1-5, wherein the cannabis composition comprises a standardized extract comprising: at least about 2.6% (w/w) THC, at least about 2.6% (w/w) CBD, at least about 0.1% (w/w) CBCA and/or CBC, and optionally 0.5% (w/w) Vitamin E.

7. The cannabis composition for use of any one of claims 1-6, wherein the cannabis composition comprises cannabis extracts enriched with CBCA and/or CBC, preferably wherein the cannabis composition enriched with CBCA and/or CBC comprises at least 1.1 times the predetermined minimal amount/concentration of CBCA and/or CBC.

8. The cannabis composition for use of any one of claims 1-7, wherein the cannabis composition comprises a content of CBCA and/or CBC between the predetermined minimal amount/concentration and a maximum amount/concentration of about 1.0g, per daily dose, preferably wherein the composition comprises CBCA and/or CBC at maximal concentration of less than 20.00%.

9. The cannabis composition for use of any one of claims 1-8, wherein the cannabis composition comprises cannabis extracts enriched with cannflavin A, preferably wherein the cannabis composition enriched with cannflavin A comprises at least 1.1 times the predetermined minimal amount/concentration of cannflavin A.

10. A method for classifying cannabis compositions as being suitable for the treatment of pain, the method comprising:
receiving a potential cannabis composition;
evaluating the content of CBCA and/or CBC in the potential cannabis composition;
classifying the composition as being suitable for the treatment of pain when the CBCA and/or CBC content is at or above a predetermined minimal amount/concentration of 575ug, per daily dose.

11. The cannabis composition for use of claim 10, wherein the predetermined minimal amount of CBCA and/or CBC is at least 800ug, per daily dose, preferably wherein the predetermined minimal amount is at least 1000ug CBCA and/or CBC, per daily dose, more preferably wherein the method further comprises evaluating the content of cannflavin A in the potential cannabis composition and classifying the composition as being suitable for the treatment of pain when the cannflavin A content is at or above a predetermined minimal amount/concentration of 50ug, per daily dose.

12. The method of classification of any one of claims 10-11, further comprising labeling the potential composition as being suitable for the treatment of pain, when the CBCA and/or CBC content is at or above the predetermined minimal amount/concentration, preferably further comprising labeling the potential composition as being suitable for the treatment of headaches, when the CBCA and cannflavin A content is at or above the predetermined minimal amount/concentration.

13. A standardized cannabis extract comprising extracts from a natural source and isolated and/or synthetic CBCA and/or CBC, wherein the extract comprises CBCA and/or CBC at a predetermined minimal amount/concentration of at least 575ug per daily dose, preferably. 800ug per daily dose, more preferably 1000ug per daily dose

14. The standardized cannabis extract of claim 13, wherein the extract further comprises cannflavin A at an amount of at least 50ug per daily dose, preferably wherein the standardized extract comprises cannflavin A and total THC at concentrations **characterized by** cannflavin A to total THC ratio of at least about 0.07%, preferably of at least 0.8%.

15. The standardized cannabis extract of any one of claims 13-14, wherein the standardized extract comprises: at least about 2.6% (w/w) total THC, at least about 2.6% (w/w) total CBD, at least about 0.1% (w/w) CBCA and/or CBC and optionally about 0.5% (w/w) Vitamin E, preferably wherein the cannabis extract is enriched with CBCA and/or CBC; and wherein the enriched cannabis extract comprises at least 1.1 times the predetermined minimal amount/concentration of CBCA and/or CBC.
